(19) Europäisches Patentamt
European Patent Office
Office européen des brevets

(11) Publication number: **0 483 403 A1**

(12) **EUROPEAN PATENT APPLICATION**

(21) Application number: **90120882.7**

(22) Date of filing: **31.10.90**

(51) Int. Cl.[5]: **C07D 401/12**, C07D 413/14, C07D 405/14, C07D 211/06, A61K 31/445

(43) Date of publication of application: **06.05.92 Bulletin 92/19**

(84) Designated Contracting States: **AT BE CH DE DK ES FR GB GR IT LI LU NL SE**

(71) Applicant: **HOECHST AKTIENGESELLSCHAFT**
**Postfach 80 03 20**
**W-6230 Frankfurt am Main 80(DE)**

(72) Inventor: **Henning, Rainer, Dr.**
**Im Höhlchen 16**
**W-6234 Hattersheim(DE)**
Inventor: **Urbach, Hansjörg, Dr.**
**Le-Lavandou-Strasse 41**
**W-6242 Kronberg(DE)**
Inventor: **Ruppert, Dieter, Dr.**
**Schreyerstrasse 30**
**W-6242 Kronberg(DE)**
Inventor: **Linz, Wolfgang, Dr.**
**Huxelrebenweg 54**
**W-6500 Mainz 42(DE)**

(54) **Derivatives of amino acids as inhibitors of renin, methods for their preparation, medicaments containing them and their use.**

(57) Renin inhibitory compounds of formula I

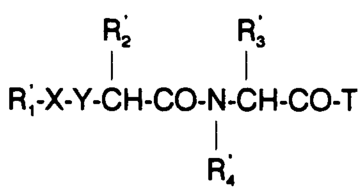

(I)

EP 0 483 403 A1

in which the residues have the following meaning:
$R_1'$ a residue of formula II or III

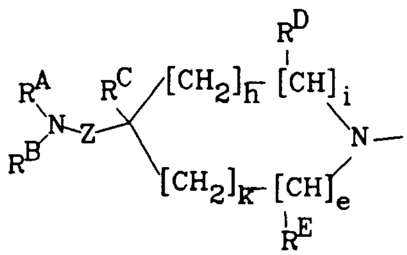

(II)

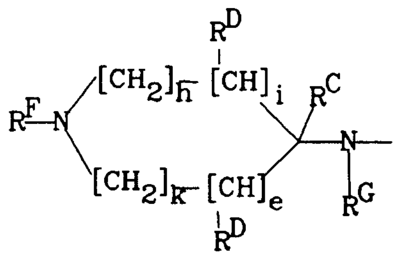

(III)

in which the residues are as defined in the specification, methods for their preparation, medicaments containing them and their use.

The following publications and patent applications and patents disclose inhibitors of renin: EP-A-184 855, EP-A-189 203, EP-A-202 531, EP-A-229 667, EP-A-309 766, EP-A-230 266, EP-A-237 202, EP-A-310 071, EP-A-310 072, WO-A-87/05 302, WO-A-88/05 050, US 4,609,643, WO-A-88/04 664, US 4,668,770, US 4,657,931, US 4,548,926, US 4,645,759, US 4,680,284, US 4,845,079, US 4,725,584, US 4,680,284, US 4,837,204, US 4,657,931, US 4,548,926, EP-A-228,192, WO-A-87/02 986; US 4,727,060; US 4,758,584; US 4,713,445; US 4,755,592; EP-A-231 919; AU 76 222 187; EP-A-310 070; EP-A-310 073; DE-A-3,721,855; US 4,705,846, EP-A-339 483; WO-A-88/02 374; WO-A-87/05 302; US 4,713,445, US 4,82,045, US 4,812,442; WO-A-87/02 581; EP-A-365,992; EP-A-364,804; Biochem. Biophys. Res. Commun. 132, 155 (1985); Biochem. Biophys. Res. Commun. 146,959 (1987); FEBS Lett. 230, 38(1988); J. Med. Chem. 30, 976 (1987); J. Med. Chem. 31, 2277 (1988).

We have now found, that compounds bearing an azacyclic acyl-aminoacyl residue on the N-terminus are highly potent and specific inhibitors of renin showing an enhanced rate of intestinal absorption and a prolonged duration of action in vivo.

In accordance with the present invention, we claim renin inhibitory compounds of formula I

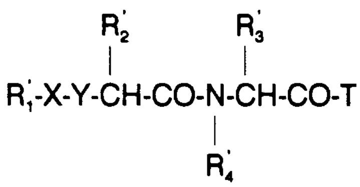

(I)

in which the residues have the following meaning:

$R_1'$ a residue of formula II or III

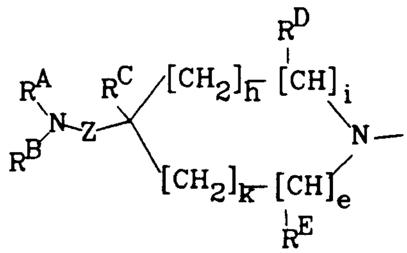

(II)

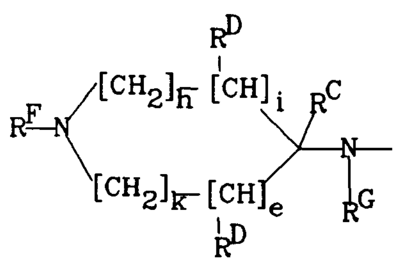

(III)

in which

$R^A$ and $R^B$ may be the same or different and are hydrogen, unsubstituted ($C_1$-$C_{21}$)-alkyl, unsubstituted or substituted ($C_3$-$C_{20}$)-Cycloalkyl, unsubstituted or substituted ($C_4$-$C_{20}$)-cycloalkyl-alkyl, substituted or unsubstituted ($C_6$-$C_{12}$)-Aryl, unsubstituted or substituted ($C_6$-$C_{12}$)-Aryl-($C_1$-$C_{20}$)-alkyl, unsubstituted or substituted ($C_5$-$C_{12}$)-hetaryl-($C_1$-$C_8$)-alkyl, or, if not covered by the definitions above are unsubstituted or substituted ($C_1$-$C_{18}$)-alkanoyl, unsubstituted or substituted ($C_3$-$C_8$)-cycloalkyl-($C_1$-$C_8$)-alkanoyl, substituted or unsubstituted ($C_7$-$C_{13}$)-Aroyl, unsubstituted or substituted ($C_5$-$C_{13}$)-heteroaroyl, unsubstituted or substituted ($C_6$-$C_{12}$)-aryl-($C_1$-$C_{18}$)-alkanoyl, unsubstituted or substituted ($C_5$-$C_{13}$)-hetaryl-($C_1$-$C_{18}$)-alkanoyl or unsubstituted or substituted ($C_8$-$C_{12}$)-aryl-($C_1$-$C_4$)-alkoxycarbonyl

or
$R^A$ and $R^B$ form together with the nitrogen atom bearing them a 4-8-numbered heterocyclic ring which may be unsaturated or saturated and which may contain another heteroatom from the group N, O or S;
or
$R^A$ is defined as above and $R^B$ is
unsubstituted or substituted ($C_1$-$C_4$)-alkylamino,
unsubstituted or substituted Di-($C_1$-$C_4$)-alkylamino, hydroxy,
unsubstituted or substituted ($C_1$-$C_4$)-alkoxy,
unsubstituted or substituted ($C_1$-$C_4$)-alkylsulfonyl,
unsubstituted or substituted ($C_6$-$C_{12}$)-arylsulfonyl or carbamidoyl;

$R^F$ has the meaning as defined above for $R^A$ and $R^B$ and may be the same or different;
$R^G$ is hydrogen, ($C_1$-$C_8$)-alkyl, ($C_3$-$C_8$)-cycloalkyl, ($C_3$-$C_8$)-cycloalkyl-($C_1$-$C_4$)-alkyl, unsubstituted or substituted ($C_6$-$C_{12}$)-aryl, unsubstituted or substituted ($C_7$-$C_{13}$)-aralkyl;
$R^C$, $R^D$ and $R^E$ may be the same or different and are hydrogen, ($C_1$-$C_6$)-alkyl or
$R^D$ and $R^E$ together form a ($C_1$-$C_4$)-alkylene bridge;
h is 0, 1, 2 or 3;
i is 0, 1, 2 or 3;
k is 1, 2, 3 or 4;
l is 1, 2, 3 or 4;
X is -CO-, -CS-, $-SO_2-$ or -SO-;
Y is $(CH_2)_q$-$(CR^HR^L)_r$-, -O- or -S-, in which
q = 0, 1, 2 or 3;
r = 0, 1 or 2;
$R^H$ and $R^L$ are the same or different and hydrogen or ($C_1$-$C_6$)-alkyl;
Z is absent or a straight chain or branched ($C_1$-$C_6$)-alkylene-residue;
$R'_2$ is hydrogen, ($C_1$-$C_{10}$)-alkyl; ($C_6$-$C_{12}$)-aryl; ($C_6$-$C_{12}$)-aryl-($C_1$-$C_4$)-alkyl; ($C_3$-$C_8$)-cycloalkyl; ($C_3$-$C_8$)-cycloalkyl-($C_1$-$C_4$)-alkyl, hetaryl or hetaryl-($C_1$-$C_4$)-alkyl, aryl and hetaryl being optionally substituted by one, two or three residues from the group halogen, hydroxy, ($C_1$-$C_4$)-alkoxy, $CF_3$, ($C_1$-$C_4$)-alkyl;
$R'_3$ is the side chain of a natural or unnatural amino acid, preferably from the group phenylalanine, histidine, tyrosine, tryptophan, methionine, leucine, isoleucine, asparagine, aspartic acid, β-2-furyl alanine, β-3-furyl alanine, lysine, ornithine, valine, alanine, 2,4-diaminobutyric acid, arginine, 4-chlorophenyl alanine, methionine sulfone, methionine sulfoxide, 2-pyridylalanine, 3-pyridylalanine, cyclohexylalanine, cyclohexylglycine, O-tert.-butyl tyrosine, phenylglycine, 1-naphthylalanine, 2-naphthylalanine, 4-nitrophenylalanine, norvaline, β-2-benzo[b]thienylalanine, β-3-benzo[b]thienylalanine, 2-fluorophenylalanine, 3-fluorophenylalanine, 4-fluorophenylalanine, norleucine, cysteine, S-methylcysteine, 1,2,3,4-tetrahydroisoquinoline-3-carboxylic acid, homophenylalanine, DOPA, O-dimethyl-DOPA, N-methyl-histidine, 2-amino-4-(2-thienyl)-butyric acid, 2-amino-4-(3-thienyl)-butyric acid, 3-(2-thienyl)-serine, 2- or 4-thiazolyl alanine, 1,3-thioxolane-2-yl-alanine, 3-(N-pyrrolyl)-alanine, 1-, 3-, or 4-pyrazolylalanine;
$R'_4$ is hydrogen or ($C_1$-$C_6$)-alkyl, preferably hydrogen and
T is a mimic of the Leu-Val cleavage site of angiotensinogen,

or a pharmaceutically acceptable salt thereof.

The term "mimic of the Leu-Val cleavage site of angiotensinogen" as used herein includes,

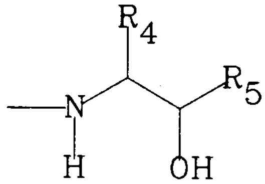

disclosed in EP-A-365 992,
wherein $R_4$ is

(I) loweralkyl,
(II) cycloalkylalkyl or
(III) arylalkyl; and

$R_5$ is

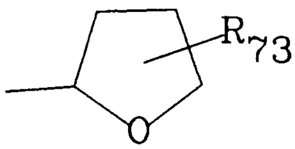

wherein $R_{73}$ is loweralkyl,

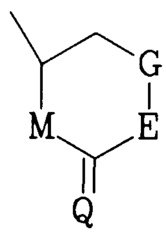

wherein

1) M is
i) O,
ii) S or
iii) NH;

2) Q is
i) O or
ii) S;

3) E is
i) O,
ii) S,
iii) $CHR_{61}$ wherein $R_{61}$ is loweralkyl,
iv) $C=CH_2$ or
v) $NR_{18}$ wherein $R_{18}$ is
a) hydrogen,
b) loweralkyl,
c) hydroxyalkyl,
d) hydroxy,
e) alkoxy,
f) amino or
g) alkylamino;

and

4) G is
i) absent,
ii) $CH_2$ or
iii) $NR_{19}$ wherein $R_{19}$ is hydrogen or loweralkyl,
with the proviso that when G is $NR_{19}$, then $R_{18}$ is loweralkyl or hydroxyalkyl,

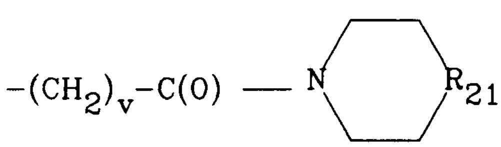

wherein
1) v is 0 or 1 and
2) $R_{21}$ is
i) NH,
ii) O,
iii) S or
iv) $SO_2$, or v) a substituted methylene group.

The term "mimic or the Leu-Val cleavage site of angiotensinogen" as used herein also includes the substituents (T) disclosed in the following references; Luly et al., U.S. Patent No. 4,645,759, issued February 24, 1987, which is hereby incorporated by reference, discloses mimics of the Leu-Val cleavage site of angiotensinogen having the formula

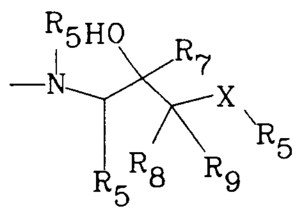

wherein $R_4$, $R_5$, $R_6$, $R_7$, $R_8$, $R_9$ and X are as defined therein;
Luly et al., U.S. Patent No. 4,652,551, issued March 24, 1987, which is hereby incorporated by reference, discloses mimics of the Leu-Val cleavage site of angiotensinogen having the formula

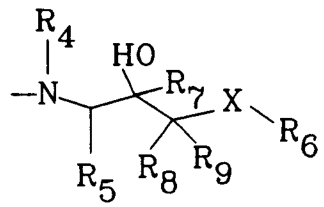

wherein $R_4$, $R_5$, $R_6$, $R_7$, $R_8$, $R_9$ and X are as defined therein;
Luly et al, U.S. Patent No. 4,680,284, issued July 14, 1987, which is hereby incorporated by reference, discloses mimic of the Leu-Val cleavage site of angiotensinogen having the formula

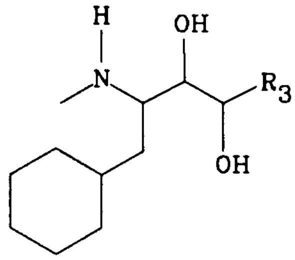

wherein $R_3$ is as defined therein;
Luly et al., U.S. Patent No. 4,725,584, issued February 16, 1988, which is hereby incorporated by reference, discloses mimics of the Leu-Val cleavage site of angiotensinogen having the formula

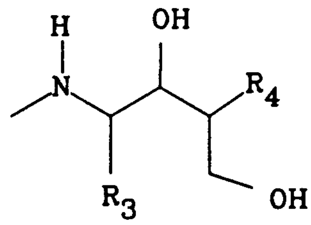

wherein $R_3$ and $R_4$ are as defined therein;

Luly et al., U.S. Patent No. 4,725,583, issued February 16, 1988, which is hereby incorporated by reference, discloses mimic of the Leu-Val cleavage site of angiotensinogen having the formula

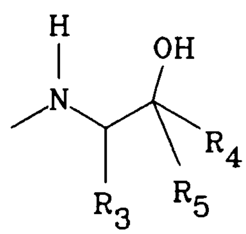

wherein $R_3$, $R_4$ and $R_5$ are as defined therein;

Rosenberg et al., U.S. Patent No. 4,837,204, issued June 6, 1989, which is hereby incorporated by reference, discloses mimics of the Leu-Val cleavage site of angiotensinogen having the formula

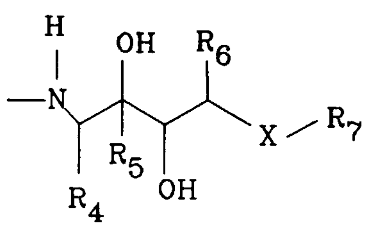

wherein $R_4$, $R_5$, $R_6$, $R_7$ and X are as defined therein;

Luly et al., U.S. Patent No. 4,845,079, issued July 4, 1989, which is hereby incorporated by reference, discloses mimics of the Leu-Val cleavage site of angiotensinogen having the formula

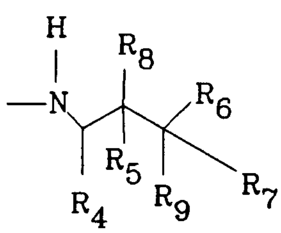

wherein $R_4$, $R_5$, $R_6$, $R_7$, $R_8$ and $R_9$ are as defined therein;

Sham, U.S. Patent No. 4,826,958, issued May 2, 1989, which is hereby incorporated by reference, discloses mimics of the Leu-Val cleavage site of angiotensinogen having the formula

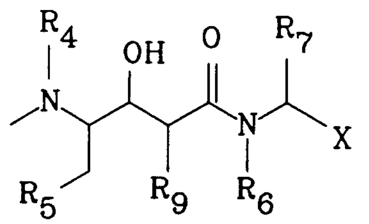

wherein $R_4$, $R_5$, $R_6$, $R_7$, $R_9$ and X are as defined therein;
Rosenberg et al., U.S. Patent No. 4,857,507, issued August 15, 1989, which is hereby incorporated by reference, discloses mimics of the Leu-Val cleavage site of angiotensinogen having the formula

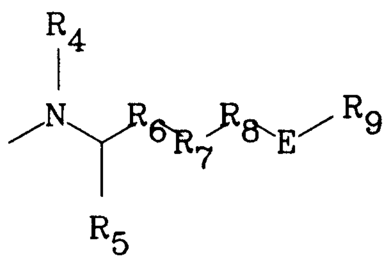

wherein $R_4$, $R_5$, $R_6$, $R_7$, $R_8$, $R_9$ and E are as defined therein;
Luly et al., U.S. Patent No. 4,826,815, issued May 2, 1989, which is hereby incorporated by reference, discloses mimics of the Leu-Val cleavage site of angiotensinogen having the formula

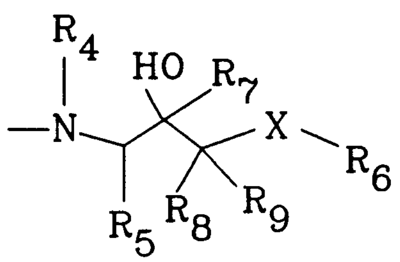

wherein $R_4$, $R_5$, $R_6$, $R_7$, $R_8$, $R_9$ and X are as defined therein;
Bender et al., U.S. Patent NO. 4,818,748, issued April 4, 1989, which is hereby incorporated by reference, discloses mimics of the Leu-Val cleavage site of angiotensinogen having the formula

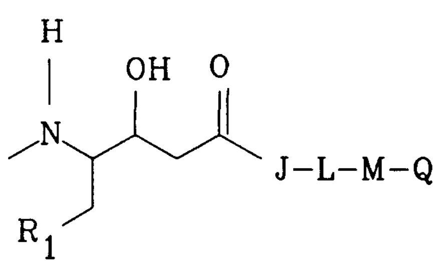

wherein $R_1$, J, L, M and Q are as defined therein;
Fuhrer et al., U.S. Patent No. 4,613,676, issued September 23, 1986, which is hereby incorporated by reference, discloses mimics of the Leu-Val cleavage site of angiotensinogen having the formula

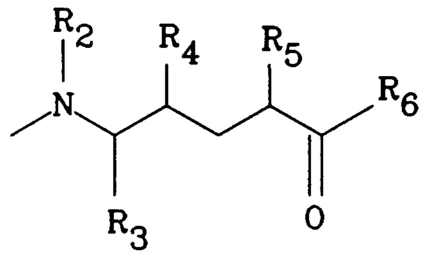

wherein $R_2$, $R_3$, $R_4$, $R_5$ and $R_6$ are as defined therein;

Riniker et al., U.S. Patent No. 4,595,677, issued June 17, 1986, which is hereby incorporated by reference, discloses mimics of the Leu-Val cleavage site of angiotensinogen having the formula

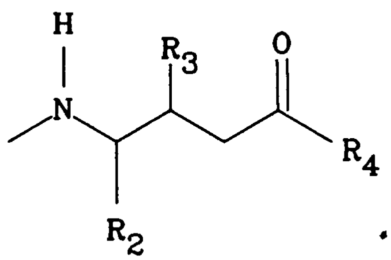

wherein $R_2$, $R_3$ and $R_4$ are as defined therein;

Buhlmayer et al., U.S. Patent No. 4,727,060, issued February 23, 1988, which is hereby incorporated by reference, discloses mimics of the Leu-Val cleavage site of angiotensinogen having the formula

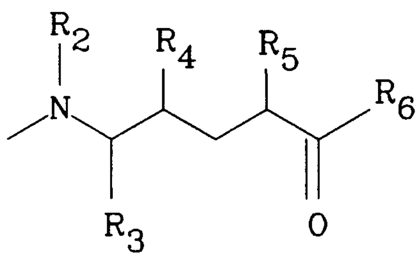

wherein, $R_2$, $R_3$, $R_4$, $R_5$ and $R_6$ are as defined therein;

Buhlmayer et al., U.S. Patent No. 4,758,584, issued July 19, 1988, which is hereby incorporated by reference, discloses mimics of the Leu-Val cleavage site of angiotensinogen having the formula

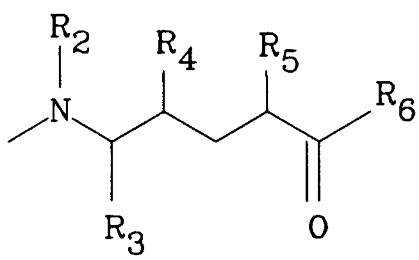

wherein $R_2$, $R_3$, $R_4$, $R_5$ and $R_6$ are as defined therein;

Szelke et al., U.S. Patent No. 4,609,643, issued September 2, 1986, which is hereby incorporated by reference, discloses mimics of the Leu-Val cleavage site of angiotensinogen having the formula

-A-B-Z-W

wherein A, B, Z and W are as defined therein;
Szelke et al., U.S. Patent No. 4,650,661, issued March 17, 1987, which is hereby incorporated by reference, discloses mimics of the Leu-Val cleavage site of angiotensinogen having the formula

-A-B-Z-W

wherein A, B, Z and W are a s defined therein;
Szelke et al., U.S. Patent No. 4,713,445, issued December 15, 1987, which is hereby incorporated by reference, discloses mimics of the Leu-Val cleavage site of angiotensinogen having the formula

-A-B-Z-W

wherein, A, B, Z and W are as defined therein;
Iizuka et al., U.S. Patent No. 4,656,269, issued April 7, 1987, which is hereby incorporated by reference, discloses mimics of the Leu-Val cleavage site of angiotensinogen having the formula

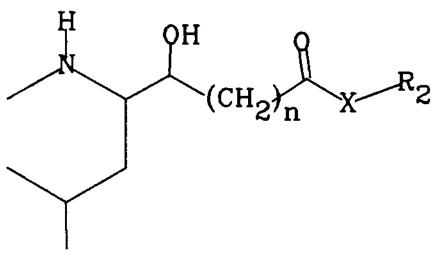

wherein n, X and $R_2$ are as defined therein;
Iizuka, et at., U.S. Patent No. 4,711,958, issued December 8, 1987, which is hereby incorporated by reference, discloses mimics of the Leu-Val cleavage site of angiotensinogen having the formula

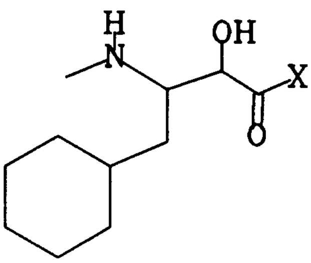

wherein X is as defined therein;
Kleinman et al., U.S. Patent No. 4,729,985, issued March 8, 1988, which is hereby incorporated by reference, discloses mimics of the Leu-Val cleavage site of angiotensinogen having the formula

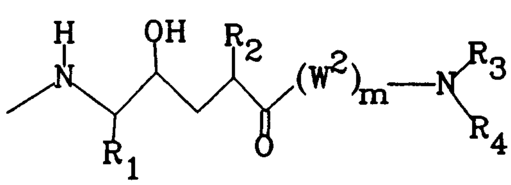

wherein $R_1$, $R_2$, m, $W^2$, $R_3$ and $R_4$ are as defined therein;

Hoover, U.S. Patent No. 4,668,769, issued May 26, 1987, which is hereby incorporated by reference, discloses mimics of the Leu-Val cleavage site of angiotensinogen having the formula

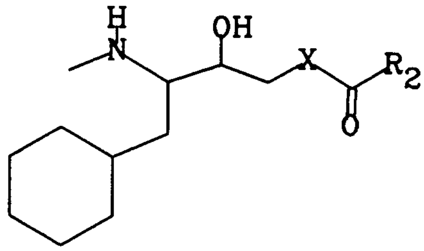

wherein X and $R_2$ are as defined therein;

Hoover et al., U.S. Patent No. 4,814,342, issued March 21, 1989, which is hereby incorporated by reference, discloses mimics of the Leu-Val cleavage site of angiotensinogen having the formula

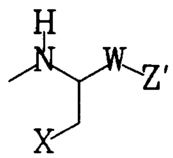

wherein X, W and Z' are as defined therein;

Bindra et al., U.S. Patent No. 4,749,687, issued June 7, 1988, which is hereby incorporated by reference, discloses mimics of the Leu-Val cleavage site of angiotensinogen having the formula

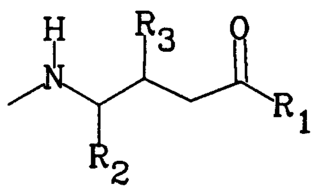

wherein $R_1$, $R_2$ and $R_3$ are as defined therein;

Hoover et al., U.S. Patent No. 4,814,342, issued March 21, 1989, which is hereby incorporated by reference, discloses mimics of the Leu-Val cleavage site of angiotensinogen having the formula

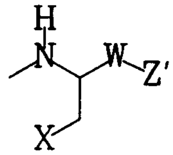

wherein X, W and Z' are as defined therein;

Matsueda et al., U.S. Patent No. 4,698,329, issued October 6, 1987, which is hereby incorporated by reference, discloses mimics of the Leu-Val cleavage site of angiotensinogen having the formula

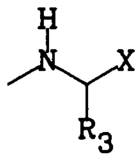

wherein $R_3$ and X are as defined therein;

Matsueda et al., U.S. Patent No. 4,548,926, issued October 22, 1985, which is hereby incorporated by reference, discloses mimics of the Leu-Val cleavage site of angiotensinogen having the formula

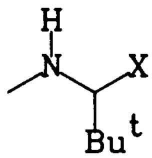

wherein $Bu^t$ and X are as defined therein;

Wagnon et al., U.S. Patent No. 4,725,580, issued February 16, 1988, which is hereby incorporated by reference, discloses mimics of the Leu-Val cleavage site of angiotensinogen having the formula

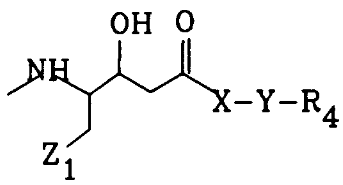

wherein $Z_1$, X, Y and $R_4$ are as defined therein;

Wagnon et al., U.S. Patent No. 4,746,648, issued May 24, 1988, which is hereby incorporated by reference, discloses mimics of the Leu-Val cleavage site of angiotensinogen having the formula

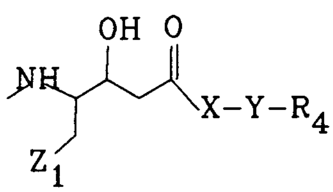

wherein $Z_1$, X, Y and $R_4$ are as defined therein;

Cazaubon et al., U.S. Patent No. 4,481,192, issued November 6, 1984, which is hereby incorporated by reference, discloses mimics of the Leu-Val cleavage site of angiotensinogen having the formula

-$Statyl_1$-Ala-$Statyl_2$-R

wherein $Statyl_1$, Ala, $Statyl_2$ and R are as defined therein;

Hansen et al., U.S. Patent No. 4,722,922, issued February 2, 1988, which is hereby incorporated by reference, discloses mimics of the Leu-Val cleavage site of angiotensinogen having the formula

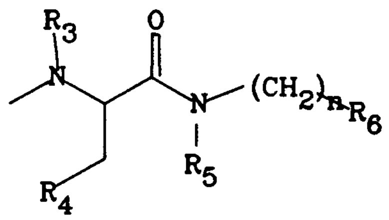

wherein $R_3$, $R_4$, $R_5$ and $R_6$ are as defined therein;

Hansen et al., U.S. Patent No. 4,510,085, issued April 9, 1985, which is hereby incorporated by reference, discloses mimics of the Leu-Val cleavage site of angiotensinogen having the formula

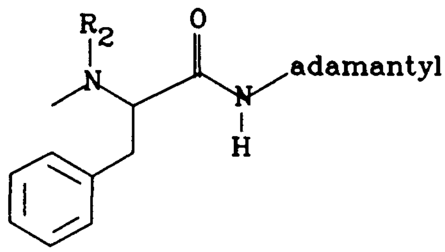

wherein $R_2$ is as defined therein;

Baren et al., U.S. Patent No. 4,657,931, issued April 14, 1987, which is hereby incorporated by reference, discloses mimics of the Leu-Val cleavage site of angiotensinogen having the formula

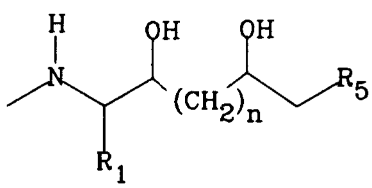

wherein $R_1$, n and $R_5$ are as defined therein;

Hansen et al., U.S. Patent No. 4,514,332, issued April 30, 1985, which is hereby incorporated by reference, discloses mimics of the Leu-Val cleavage site of angiotensinogen having the formula

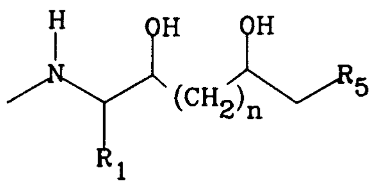

Natarajan et al., U.S. Patent No. 4,757,050, issued July 12, 1988, which is hereby incorporated by reference, discloses mimics of the Leu-Val cleavage site of angiotensinogen having the formula

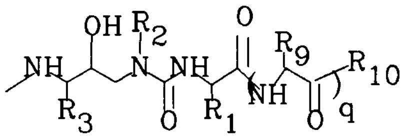

wherein $R_1$, $R_2$, $R_3$, q, $R_9$ and $R_{10}$ are as defined therein;

Gordon, U.S. Patent No. 4,749,781, issued June 7, 1988, which is hereby incorporated by reference, discloses mimics of the Leu-Val cleavage site of angiotensinogen having the formula

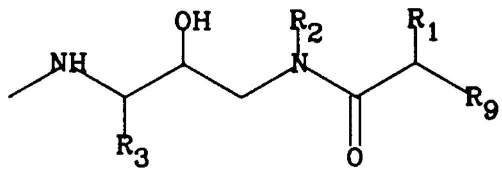

wherein $R_1$, $R_2$, $R_3$ and $R_9$ are as defined therein;

Ryono et al., U.S. Patent No. 4,665,193, issued May 12, 1987, which is hereby incorporated by reference, discloses mimics of the Leu-Val cleavage site of angiotensinogen having the formula

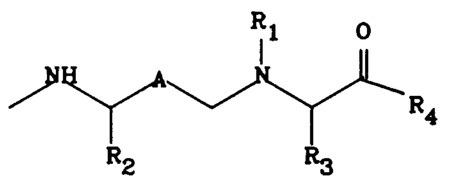

wherein $R_1$, $R_2$, $R_3$, $R_4$ and A are as defined therein;

Ryono et al., U.S. Patent No. 4,616,088, issued October 7, 1986, which is hereby incorporated by reference, discloses mimics of the Leu-Val cleavage site of angiotensinogen having the formula

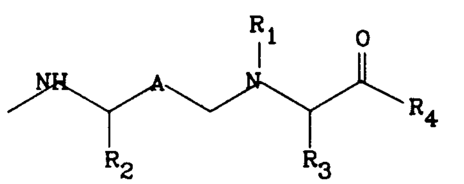

wherein $R_1$, $R_2$, $R_3$, $R_4$ and A are as defined therein;

Ryono et al., U.S. Patent No. 4,629,724, issued December 16, 1986, which is hereby incorporated by reference, discloses mimics of the Leu-Val cleavage site of angiotensinogen having the formula

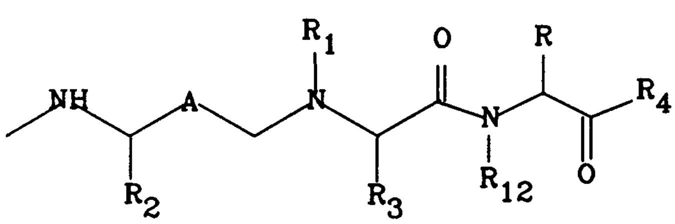

wherein $R_1$, $R_2$, $R_3$, $R_4$, R, $R_{12}$ and A are as defined therein;

Patel, U.S. Patent No. 4,820,691, issued April 11, 1989, which is hereby incorporated by reference, discloses mimics of the Leu-Val cleavage site of angiotensinogen having the formula

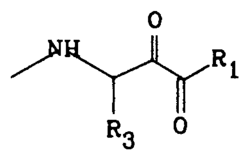

wherein $R_1$ and $R_3$ are as defined therein;

Thaisrivongs, U.S. Patent No. 4,705,846, issued November 10, 1987, which is hereby incorporated by reference, discloses mimics of the Leu-Val cleavage site of angiotensinogen having the formula

$-E_{10}-F_{11}-G_{12}-H_{13}-I_{14}-Z$

wherein $E_{10}$, $F_{11}$, $G_{12}$, $H_{13}$, $I_{14}$ and Z are as defined therein;

Hudspeth et al., U.S. Patent No. 4,743,585, issued May 10, 1988, which is hereby incorporated by reference, discloses mimics of the Leu-Val cleavage site of angiotensinogen having the formula

$-T-(C)_n-W-(D)_n-V-(E)_n-U$

wherein T, C, W, D, V, E, U and n are as defined therein;

Hudspeth et al., U.S. Patent No. 4,735,933, issued April 5, 1988, which is hereby incorporated by reference, discloses mimics of the Leu-Val cleavage site of angiotensinogen having the formula

-Y-W-U

wherein Y W and U are as defined therein;

Kaltenbronn et al., U.S. Patent No. 4,804,743, issued February 14, 1989, which is hereby incorporated by reference, discloses mimics of the Leu-Val cleavage site of angiotensinogen having the formula

-T-U-V-W

wherein T, U, V and W are as defined therein;

Pinori et al., U.S. Patent No. 4,560,505, issued December 24, 1985, which is hereby incorporated by reference, discloses mimics of the Leu-Val cleavage site of angiotensinogen having the formula

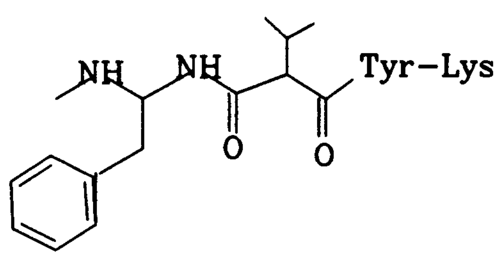

wherein Tyr and Lys are as defined therein;

Yamato et al., U.S. Patent No. 4,683,220, issued July 28, 1987, which is hereby incorporated by reference, discloses mimics of the Leu-Val cleavage site of angiotensinogen having the formula

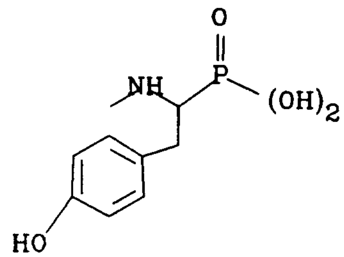

Boger et al., U.S. Patent No. 4,668,770, issued May 26, 1987, which is hereby incorporated by reference, discloses mimics of the Leu-Val cleavage site of angiotensinogen having the formula

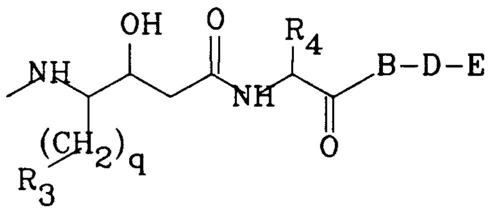

wherein $R_3$, $R_4$, q, B, D and E are as defined therein;

Boger, U.S. Patent No. 4,668,663, issued May 26, 1987, which is hereby incorporated by reference, discloses mimics of the Leu-Val cleavage site of angiotensinogen having the formula

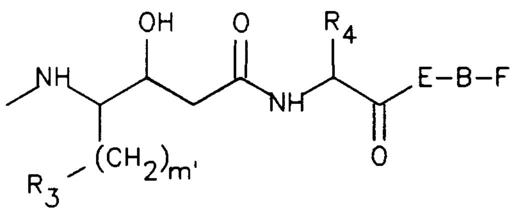

wherein $R_3$, $R_4$, m', E, B and F are as defined therein;

Bock et al., U.S. Patent No. 4,636,491, issued January 13, 1987, which is hereby incorporated by reference, discloses mimics of the Leu-Val cleavage site of angiotensinogen having the formula

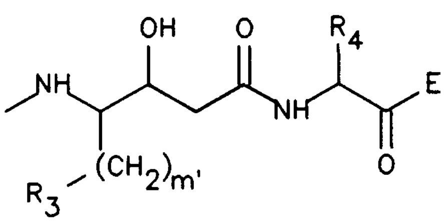

wherein $R_3$, $R_4$, m' and E are as defined therein;

Bock et al., U.S. Patent No. 4,683,310, issued May 5, 1987, which is hereby incorporated by reference, discloses mimics of the Leu-Val cleavage site of angiotensinogen having the formula

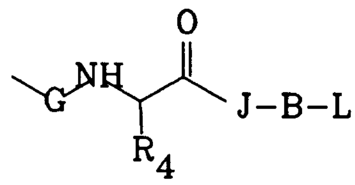

wherein G, $R_4$, J, B and L are as defined therein;

Boger et al., U.S. Patent No. 4,661,473, issued April 28, 1987, which is hereby incorporated by reference, discloses mimics of the Leu-Val cleavage site of angiotensinogen having the formula

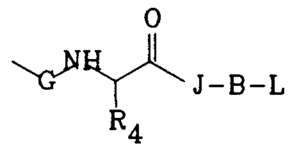

wherein G, $R_4$, J, B and L are as defined therein;

Veber et al., U.S. Patent No. 4,479,941, issued October 30, 1984, which is hereby incorporated by reference, discloses mimics of the Leu-Val cleavage site of angiotensinogen having the formula

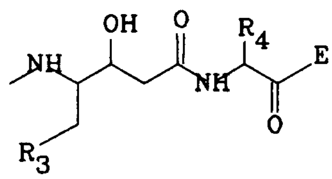

wherein $R_3$, $R_4$ and E are as defined therein;

Boger et al., U.S. Patent No. 4,470,971, issued September 11, 1984, which is hereby incorporated by reference, discloses mimics of the Leu-Val cleavage site of angiotensinogen having the formula

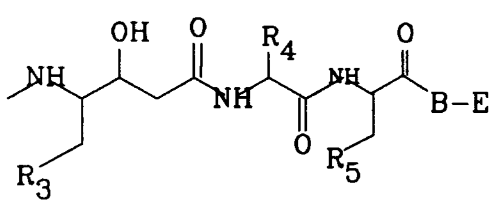

wherein $R_3$, $R_4$, $R_5$, B and E are as defined therein;

Veber et al., U.S. Patent No. 4,384,994, issued May 24, 1983, which is hereby incorporated by reference, discloses mimics of the Leu-Val cleavage site of angiotensinogen having the formula

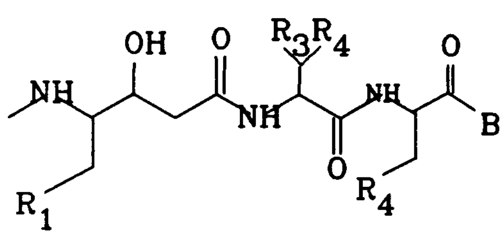

wherein $R_1$, $R_2$, $R_3$, $R_4$ and B are as defined therein;

Boger et al., U.S. Patent No. 4,812,442, issued March 14, 1989, which is hereby incorporated by reference, discloses mimics of the Leu-Val cleavage site of angiotensinogen having the formula

-G-J

wherein G and J are as defined therein;

Evans, U.S. Patent No. 4,665,055, issued May 12, 1987, which is hereby incorporated by reference, discloses mimics of the Leu-Val cleavage site of angiotensinogen having the formula

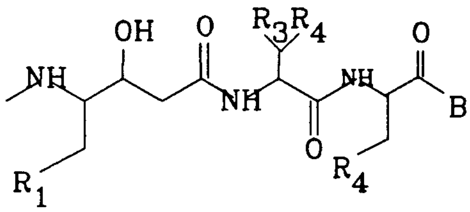

wherein $R_4$, $R_5$, B and C are as defined therein;

Evans et al., U.S. Patent No. 4,609,641, issued September 2, 1986, which is hereby incorporated by reference, discloses mimics of the Leu-Val cleavage site of angiotensinogen having the formula

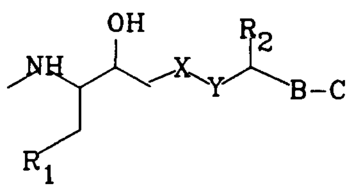

wherein $R_1$, $R_2$, X, Y, B and C are as defined therein;

Patchett et al., U.S. Patent No. 4,839,357, issued June 13, 1989, which is hereby incorporated by reference, discloses mimics of the Leu-Val cleavage site of angiotensinogen having the formula

-G-J

wherein G and J are as defined therein;

Boger et al., U.S. Patent No. 4,812,442, issued March 14, 1989, which is hereby incorporated by reference, discloses mimics of the Leu-Val cleavage site of angiotensinogen having the formula

-G-J

wherein G and J are as defined therein;

Boger, U.S. Patent No. 4,665,052, issued May 12, 1987, which is hereby incorporated by reference, discloses mimics of the Leu-Val cleavage site of angiotensinogen having the formula

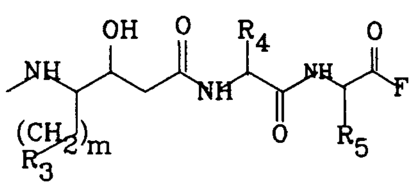

wherein $R_3$, $R_4$, $R_5$, m and F are as defined therein;

Veber et al., U.S. Patent No. 4,478,826, issued October 23, 1984, which is hereby incorporated by reference, discloses mimics of the Leu-Val cleavage site of angiotensinogen having the formula

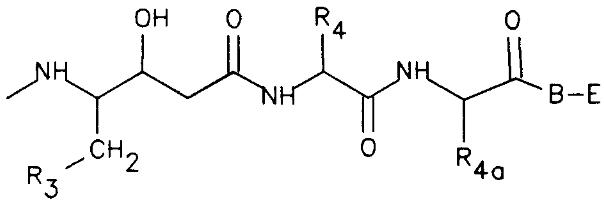

wherein $R_3$, $R_4$, $R_{4a}$, B and E are as defined therein;

Boger et al., U.S. Patent No. 4,485,099, issued November 27, 1984, which is hereby incorporated by reference, discloses mimics of the Leu-Val cleavage site of angiotensinogen having the formula

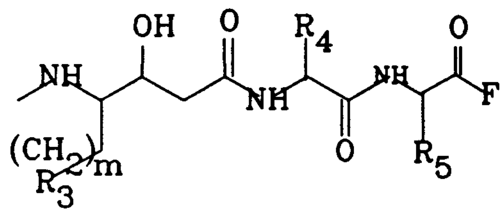

wherein $R_3$, $R_4$, $R_5$, m and F are as defined therein;

Boger et al., U.S. Patent No. 4,477,440, issued October 16, 1984, which is hereby incorporated by reference, discloses mimics of the Leu-Val cleavage site of angiotensinogen having the formula

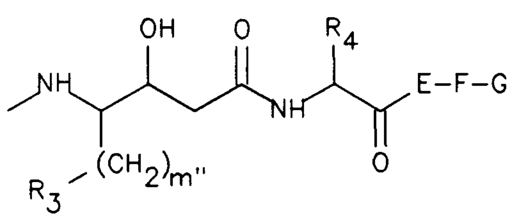

wherein $R_3$, $R_4$, m'', E, F and G are as defined therein;

Raddatz et al., U.S. Patent No. 4,721,776, issued January 26, 1988, which is hereby incorporated by reference, discloses mimics of the Leu-Val cleavage site of angiotensinogen having the formula

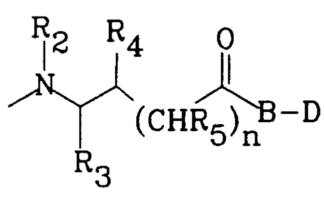

wherein $R_2$, $R_3$, $R_4$, $R_5$, n, B and D are as defined therein;

Holzemann et al., U.S. Patent No. 4,709,010, issued November 24, 1987, which is hereby incorporated by reference, discloses mimics of the Leu-Val cleavage site of angiotensinogen having the formula

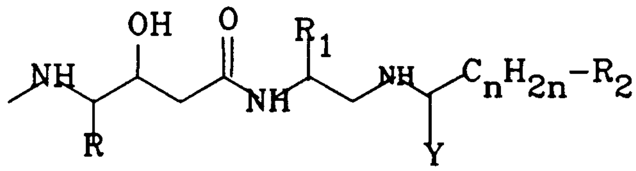

wherein R, $R_1$, $R_2$, n and Y are as defined therein;

Raddatz et al., U.S. Patent No. 4,812,555, issued March 14, 1989, which is hereby incorporated by reference, discloses mimics of the Leu-Val cleavage site of angiotensinogen having the formula

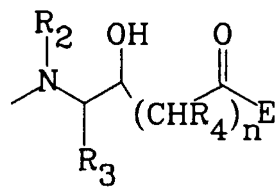

wherein $R_2$, $R_3$, $R_4$, n and E are as defined therein;

Raddatz et al., U.S. Patent No. 4,755,592, issued July 5, 1988, which is hereby incorporated by reference, discloses mimics of the Leu-Val cleavage site of angiotensinogen having the formula

-W-E-W'-Y

wherein W, E, W' and Y are as defined therein;

Raddatz et al., U.S. Patent No. 4,666,888, issued May 19, 1987, which is hereby incorporated by reference, discloses mimics of the Leu-Val cleavage site of angiotensinogen having the formula

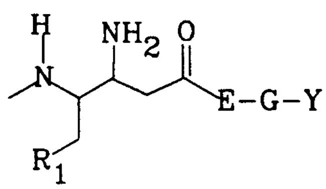

wherein $R_1$, E, G and Y are as defined therein;

Wagnon , et al., U.S. Patent No. 4,840,935, issued June 20, 1989, which is hereby incorporated by reference, discloses mimics of the Leu-Val cleavage site of angiotensinogen having the formula

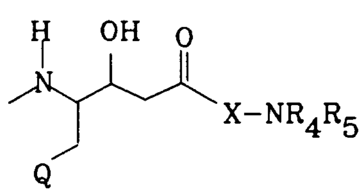

Iizuka et al., U.S. Patent No. 4,841,067, issued June 20, 1989, which is hereby incorporated by reference, discloses mimics of the Leu-Val cleavage site of angiotensinogen having the formula

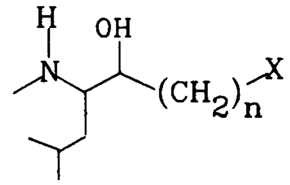

wherein n and X are as defined therein;

Raddatz et al., U.S. Patent No. 4,829,053, issued May 9, 1989, which is hereby incorporated by reference, discloses mimics of the Leu-Val cleavage site of angiotensinogen having the formula

$-N(R_2)-CH(R_3)-CR_4-(CHR_5)_n-C(O)-E-N(R_6)-(CH(R_7))_s-D$

wherein n, s, $R_2$, $R_3$, $R_4$, $R_5$, $R_6$, $R_7$, E and D are as defined therein;

European Patent Application No. EP 0 264 106, published April 20, 1988, discloses mimics of the Leu-Val cleavage site of angiotensinogen having the formula

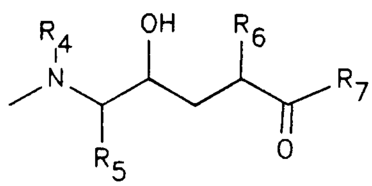

wherein $R_4$, $R_5$, $R_6$ and $R_7$ are as defined therein including $R_4$ is hydrogen or loweralkyl; $R_5$ is hydrogen, loweralkyl or an amino acid residue; $R_6$ is loweralkyl, cycloalkyl, cycloalkylalkyl or arylalkyl and $R_7$ is hydroxy, alkoxy, substituted alkoxy, amino, substituted amino or an N-heterocycle;

European Patent Application No. EP 0 272 583, published June 28, 1988, discloses mimics of the Leu-Val cleavage site of angiotensinogen having the formula

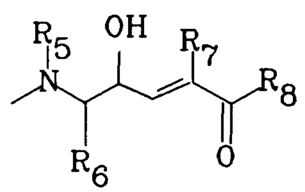

wherein $R_5$, $R_6$, $R_7$ and $R_8$ are as defined therein including $R_5$ is hydrogen or loweralkyl; $R_6$ is hydrogen, loweralkyl, cycloalkyl, cycloalkylalkyl, aryl, arylalkyl or an amino acid residue; and $R_7$ and $R_8$ are independently selected from hydrogen, loweralkyl, cycloalkyl, cycloalkylalkyl or arylalkyl;

European Patent Application No. EP 0 309 766, published April 5, 1989, discloses mimics of the Leu-Val cleavage site of angiotensinogen having the formula

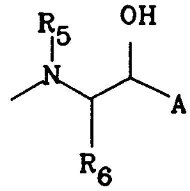

wherein $R_5$, $R_6$ and A are as defined therein including $R_5$ is hydrogen or loweralkyl; $R_6$ is hydrogen, loweralkyl, cycloalkyl, cycloalkylalkyl, aryl, aralkyl or heterocyclic; and A is - CH(OH)-$(CH_q)$-$R_7$ wherein q is 0-5 and $R_7$ is hydrogen, loweralkyl, cycloalkyl, cycloalkylalkyl, aryl, arylalkyl, substituted thioalkyl, substituted sulfone, substituted sulfoxide, substituted amino, quaternized amine, heterocyclic, carboxyalkyl, alkoxycarbonylalkyl or amidoalkyl;

European Patent Application No. EP 0 300 189, published January 25, 1989, discloses mimics of the Leu-Val cleavage site of angiotensinogen having the formula

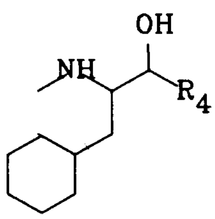

wherein $R_4$ is as defined therein including $R_4$ is loweralkyl;

European Patent Application No. EP 0 283 970, published September 28, 1988, discloses mimics of the Leu-Val cleavage site of angiotensinogen having the formula

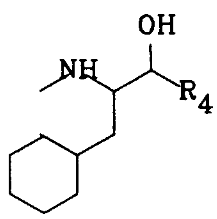

wherein $R_4$ is as defined therein including $R_4$ is loweralkyl;

European Patent Application No. EP 0 255 082, published February 3, 1988, discloses mimics of the Leu-Val cleavage site of angiotensinogen having the formula

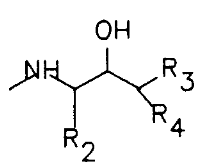

wherein $R_2$, $R_3$ and $R_4$ are as defined therein including $R_2$ is hydrogen, alkyl, cycloalkyl, cycloalkylalkyl, aryl or arylalkyl; $R_3$ is hydrogen, alkyl or arylalkyl; and $R_4$ is -X-$(CH_2)_n$-$R_7$ wherein X is absent, O or S, n is 0-4 and $R_7$ is hydrogen, hydroxy, amino, heteroaryl or - CH($R_9$)-$(CH_2)_p$-Y-$(CH_2)_q$-$R_{10}$ wherein p, q, Y and $R_{10}$ are as defined therein;

European Patent Application No. EP 0 230 242, published July 29, 1987, discloses mimics of the Leu-Val cleavage site of angiotensinogen having the formula

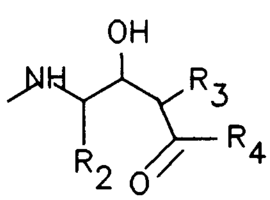

wherein $R_2$, $R_3$ and $R_4$ are as defined therein including $R_2$ is hydrogen, alkyl, cycloalkyl, aryl or arylalkyl; $R_3$ is hydrogen, alkyl or alkenyl; and $R_4$ is $-N(R_5)-CH(R_6)-(CH_2)_n-Ar$ or $-N(R_5)-CH(R_6)-CH=CH-(CH_2)_m-Ar$ wherein n is 0-6, m is 0-4, $R_5$ is hydrogen or alkyl and $R_6$ is hydrogen, alkyl, hydroxyalkyl, alkoxyalkyl, thioalkoxyalkyl, carboxyalkyl, alkoxycarbonylalkyl, haloalkyl, alkylaminoalkyl, alkoxycarbonylaminoalkyl or arylalkoxycarbonylaminoalkyl;

European Patent Application No. EP 0 310 015, published April 5, 1989, discloses mimics of the Leu-Val cleavage site of angiotensinogen having the formula

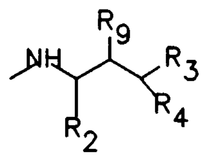

wherein $R_2$, $R_3$, $R_4$ and $R_9$ are as defined therein including $R_2$ is hydrogen, alkyl, cycloalkyl, cycloalkylalkyl, aryl or arylalkyl; $R_3$ is hydrogen, alkyl, aryl or arylalkyl, $R_9$ is hydroxy or fluoro; and $R_4$ is $-(CH_2)_p-X-(CH_2)_q-R_7$ wherein p is 04, X is $-CF_2-$, -C(O)- or $-CH(R_8)-$ wherein $R_8$ is alkyl, alkoxy, thioalkoxy, alkylamino, hydroxy, azido or halo and $R_7$ is hydrogen, hydroxy, amino, aryl or heteroaryl;

European Patent Application No. EP 0 315 574, published May 10, 1989, discloses mimics of the Leu-Val cleavage site of angiotensinogen having the formula

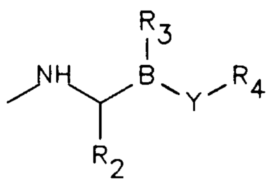

(B is a boron atom)

wherein $R_2$, X, Y, $R_3$ and $R_4$ are as defined therein including $R_2$ is hydrogen, alkyl, cycloalkyl, cycloalkylalkyl, aryl or heterocyclic; X and Y are independently selected from O or $-N(R_{13})$-wherein $R_{13}$ is hydrogen, alkyl or substituted alkyl; and $R_3$ and $R_4$ are independently selected from hydrogen, alkyl or aryl; or the boron containing substituent is a boron containing cyclic group;

Japanese Patent Application No. J 63 275 552, published November 14, 1988 discloses mimics of the Leu-Val cleavage site of angiotensinogen having the formula

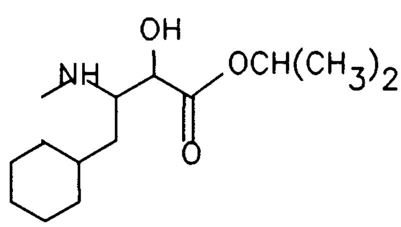

European Patent Application No. EP 0 252 727, published January 13, 1988, discloses mimics of the Leu-Val cleavage site of angiotensinogen having the formula

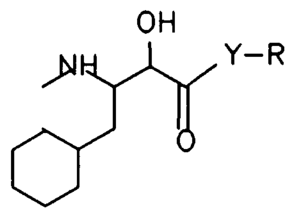

wherein Y and R are as defined therein including Y is O or NH and R is alkyl, cycloalkyl or halogenated alkyl;

European Patent Application No. EP 0 244 083, published November 4, 1987, discloses mimics of the Leu-Val cleavage site of angiotensinogen having the formula

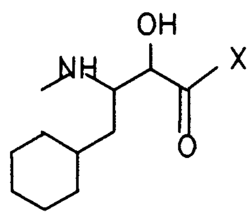

wherein X is as defined therein including X is alkoxy, alkylamino, cycloalkyloxy, morpholino and haloalkoxy.

European Patent Application No. EP 0 216 539, published April 1, 1987, discloses mimics of the Leu-Val cleavage site of angiotensinogen having the formula

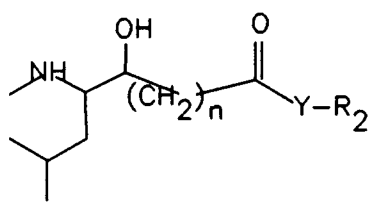

wherein n, Y and $R_2$ are as defined therein including n is 0-1, Y is O or NH and $R_2$ is alkyl;

European Patent Application No. EP 0 206 807, published December 30, 1986, discloses mimics of the Leu-Val cleavage site of angiotensinogen having the formula

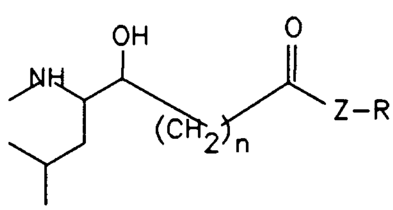

wherein n, Z and R are as defined therein including n is 0-1, Z or O or NH and R is alkyl;

European Patent Application No. EP 0 190 891, published August 13, 1986, discloses mimics of the Leu-Val cleavage site of angiotensinogen having the formula

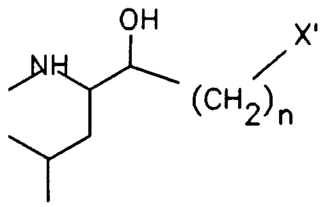

wherein n and X' are as defined therein including n is 0-1 and X' is alkoxycarbonyl, aralkoxyarbonyl, or -C-(O)$NR_1R_2$ wherein $R_1$ is hydrogen, alkyl or aralkyl and $R_2$ is alkyl or $-CH_2$-Y-R wherein Y is O or NH and R is alkyl or aralkyl;
European Patent Application No. EP 0 181 110, published May 14, 1986, discloses mimics of the Leu-Val cleavage site of angiotensinogen having the formula

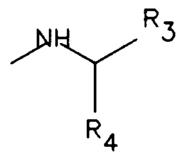

wherein $R_3$ and $R_4$ are as defined therein including $R_3$ is -CHO or $-CH_2OH$ and $R_4$ is isobutyl or benzyl;
European Patent Application No. EP 0 297 816, published January 4, 1989, discloses mimics of the Leu-Val cleavage site of angiotensinogen having the formula

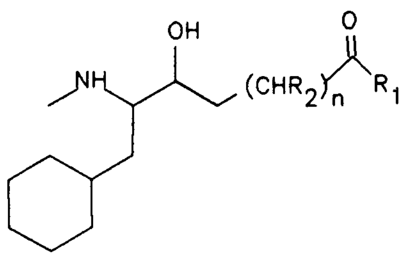

wherein n, $R_1$ and $R_2$ are as defined therein including n is 0-1, $R_1$ is $-NH_2$, alkylamino, alkoxy, or 2-alkoxycarbonylpyrrolidin-1-yl and $R_2$ is hydrogen, alkyl, alkenyl, haloalkenyl or azide substituted alkenyl;
European Patent Application No. EP 0 297 815, published January 4, 1989, discloses mimics of the Leu-Val cleavage site of angiotensinogen having the formula

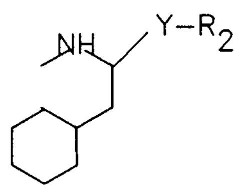

wherein Y and $R_2$ are as defined therein including Y is -CH(OH)- or -C(O)- and $R_2$ is $-CF_2C(O)NHCH_3$, $-CF_3$ or $-CF_2C(CH_2CH(CH_3)_2)COC_2H_5$;
European Patent Application No. EP 0 212 903, published March 4, 1987, discloses mimics of the Leu-Val cleavage site of angiotensinogen having the formula

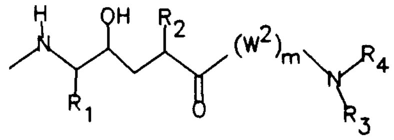

wherein m, $R_1$, $R_2$, $R_3$, $R_4$ and $W^2$ are as defined therein including m is 0-1, $R_1$ and $R_2$ are independently selected from hydrogen, alkyl, alkenyl, phenyl, naphthyl, cycloalkyl, cycloalkenyl, phenylalkyl, naphthylalkyl, cycloalkylalkyl and cycloalkenylalkyl, $R_3$ and $R_4$ are independently selected from alkyl, phenyl, naphthyl, cycloalkyl, adamantyl, phenylalkyl, naphthylalkyl, cycloalkylalkyl and adamantylalkyl; or $R_3$ is hydrogen and $R_4$ is $-CH(R_7)(CH_2)_p(Q)_rCH(R_8)(CH_2)_q-Y$ wherein p and q are independently selected from 0, 1, 2, 3, 4, 5 and 6, r is 0-1, Q is $-CH_2-$, -CH=CH-, -O-,-NH-, -CH(OH)- or -C(O)-, Y is methyl, phenyl, $-C(O)OR_9$, $-C(O)-NR_9R_{10}-$, $-C(O)NHC(O)OCH_2C_6H_5$, $-NH_2$, $-NHC(O)CH_2C_6H_5$, $-NHCH(CH_2C_6H_5)C(O)OR_9$ or $-NHCH-(CH_2C_6H_5)C(O)-NR_9R_{10}$ wherein $R_9$ and $R_{10}$ are independently selected from hydrogen, alkyl, phenyl, cycloalkyl, phenylalkyl, cycloalkylalkyl or adamantyl, and $R_7$ and $R_8$ are independently selected from hydrogen, alkyl, phenyl, cycloalkyl, phenylalkyl, cycloalkylalkyl or adamantyl; or $R_3$ and $R_4$ taken together with the nitrogen to which they are attached form a pyrrole, indoline, isoindoline, piperidine, 1,2,3,4-tetrahydroquinoline, 1,2,3,4-tetrahydroisoquinoline, perhydroazepine or morpholine ring; and $W^2$ is $-NHCH(-(CH_2)_3R_6)-C(O)-$ wherein $R_6$ is $-NH_2$, $-NHC(=NH)NH_2$ or $-CH_2NH_2$;

PCT Patent Application No. WO 88/03022, published May 5, 1988, discloses mimics of the Leu-Val cleavage site of angiotensinogen having the formula

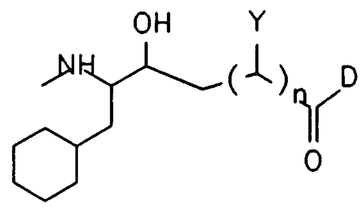

wherein n, Y and D are as defined therein including n is 0-1, Y is isobutyl, allyl or benzyl and D is 2-carboxypyrrolidin-1-yl or -ZR wherein Z is O or NH and R is alkyl, phenyl or substituted alkyl or substituted phenyl;

German Patent Application No. DE 37 25 137, published August 6, 1986, discloses mimics of the Leu-Val cleavage site of angiotensinogen having the formula

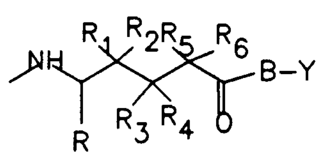

wherein R, $R_1$, $R_2$, $R_3$, $R_4$, $R_5$, $R_6$, B and Y are as defined therein including R is hydrogen, alkyl, cycloalkyl, cycloalkylalkyl, aryl, arylalkyl, heteroaryl or heterorylalkyl, $R_1$ is hydroxy, alkoxy or aryloxy, $R_2$ is hydrogen or $R_1$ and $R_2$ taken together is oxo (=O), $R_3$, $R_4$, $R_5$ and $R_6$ are independently selected from hydrogen, fluoro, chloro, alkyl, cycloalkyl, cycloalkylalkyl, aryl, arylalkyl, heteroaryl and heteroarylalkyl, B is a peptide chain containing from 1 to 10 amino acid residues and Y is hydroxy or protecting group for the peptide carboxy group;

British Patent Application No. GB 2 203 740, published October 26, 1988, discloses mimics of the Leu-Val cleavage site of angiotensinogen having the formula

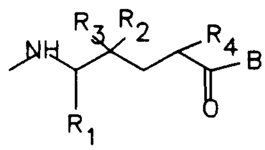

wherein $R_1$, $R_2$, $R_3$, $R_4$ and B are as defined therein including $R_1$ is a hydrophobic or hydrophilic side chain, $R_2$ is hydroxy or amino, $R_3$ is hydrogen or $R_2$ and $R_3$ taken together is oxo (=O), $R_4$ is a hydrophobic or hydrophilic side chain and B is $-NHCH(R_6)C(R_7)(R_8)C(R_9)(R_{10})CH_2C(O)NR_{11}R_{12}$ wherein $R_6$ is $R_1$, $R_7$ and $R_8$ are the same as $R_2$ and $R_3$, $R_9$ and $R_{10}$ are independently selected from hydrogen and fluoro and $R_{11}$ and $R_{12}$ are independently selected from hydrogen, alkyl, arylalkyl, heteroarylalkyl and $-CH(R_{13})C(O)R_{14}$ wherein $R_{13}$ is alkyl or hydroxyalkyl and $R_{14}$ is hydroxy, alkoxy, amino, alkylamino, aminomethylpyridyl or benzyl;

British Patent Application No. GB 2 200 115, published July 27, 1988, discloses mimics of the Leu-Val cleavage site of angiotensinogen having the formula

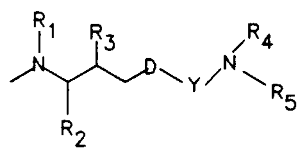

$R_1$, $R_2$, $R_3$, $R_4$, $R_5$, D and Y are as defined therein including $R_1$ is hydrogen or alkyl, $R_2$ is an amino acid side chain, $R_3$ is hydrogen, hydroxy, aryloxy or amino, $R_4$ and $R_5$ are indepenently selected from hydrogen, alkyl, arylalkyl, heterorylalkyl and $-CH(R_{12})C(O)R_{13}$ wherein $R_{12}$ is alkyl or hydroxyalkyl and $R_{13}$ is hydroxy, alkoxy, amino, alkylamino, aminomethylpyridyl or benzyl, or $-NR_4R_5$ represents pyrrolidinyl, piperidinyl, morpholinyl, piperazinyl or substituted piperazinyl; D is a bond, O, $-N(R_1)-$ or $-CH(R_1)-$ and Y is $-C(O)-$, $-S(O)_2-$ or $-P(O)-$;

German Patent Application No. DE 38 30 825, published March 23, 1989, discloses mimics of the Leu-Val cleavage site of angiotensinogen having the formula

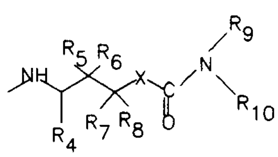

wherein $R_4$, $R_5$, $R_6$, $R_7$, $R_8$, $R_9$, $R_{10}$ and X are as defined therein including $R_4$ is a hydrophilic or hydrophobic amino acid side chain, $R_5$ is hydroxy or amino, $R_6$ is hydrogen or $R_5$ and $R_6$ taken together are oxo (=O), $R_7$ and $R_8$ are independently selected from hydrogen and fluoro, $R_9$ and $R_{10}$ are independently selected from hydrogen, alkyl and $-CH(R_{11})C(O)R_{12}$ wherein $R_{11}$ is alkyl or hydroxyalkyl and $R_{12}$ is hydroxy, alkoxy, amino, alkylamino, aminomethylpyridyl, benzyl or $-NH-(CH_2CH_2O)_m-R_1$ wherein m is 1-20 and $R_1$ is as defined therein; and X is a bond or O, NH or $-C(R_{13})(R_{14})-$ wherein $R_{13}$ and $R_{14}$ are independently selected from hydrogen, fluoro or $R_4$;

Japanese Patent Application No. J 62 246 546, published October 27, 1987, discloses mimics of the Leu-Val cleavage site of angiotensinogen having the formula

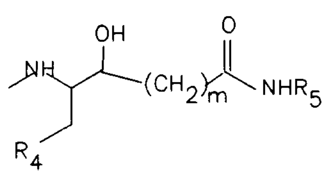

wherein m, $R_4$ and $R_5$ are as defined therein including m is 0-1, $R_4$ is alkyl, cycloalkyl or phenyl and $R_5$ is alkyl or substituted alkyl as defined therein;
European Patent Application No. EP 0 274 259, published July 13, 1988, discloses mimics of the Leu-Val cleavage site of angiotensinogen having the formula

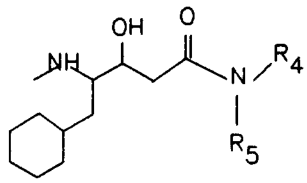

wherein $R_4$ and $R_5$ are as defined therein including $R_4$ is alkyl, hydroxyalkyl, (heterocyclic)akyl, aminoalkyl, alkylaminoalkyl or dialkylaminoalkyl and $R_5$ is hydrogen or alkyl;
European Patent Application No. EP 0 228 192, published July 8, 1987, discloses mimics of the Leu-Val cleavage site of angiotensinogen having the formula

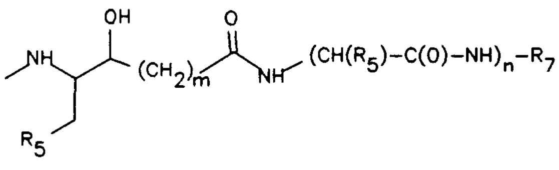

wherein m, n, $R_5$, $R_6$ and $R_7$ are as defined therein including m and n are independently selected from 0 and 1, $R_5$ is alkyl, cycloalkyl or phenyl, $R_6$ is alkyl and $R_7$ is alkyl or substituted alkyl as defined therein;
European Patent Application No. EP 0 273 893, published July 6, 1988, discloses mimics of the Leu-Val cleavage site of angiotensinogen having the formula

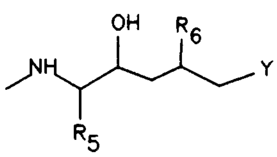

wherein $R_5$, $R_6$ and Y are as defined therein including $R_5$ is alkyl or cycloalkyl, $R_6$ is hydrogen or alkyl and Y is $-SCH(CH_3)_2$ or $-S(O)_2CH(CH_3)_2$;
European Patent Application No. EP 0 310 070, published April 5, 1989, discloses mimics of the Leu-Val cleavage site of angiotensinogen having the formula

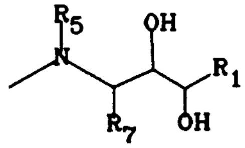

wherein $R_1$, $R_5$ and $R_7$ are as defined therein including $R_1$ is hydrogen, alkyl, haloalkyl, alkylcycloalkyl, alkylcycloalkenyl or alkoxycarbonyl, $R_5$ is hydrogen or alkyl and $R_7$ is cycloalkyl, phenyl, cycloalkylalkyl or phenylalkyl;
European Patent Application No. EP 0 310 071, published April 5, 1989 discloses mimics of the Leu-Val cleavage site of angiotensinogen having the formula

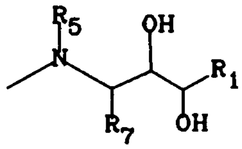

wherein $R_1$, $R_5$ and $R_7$ are as defined therein including $R_1$ is hydrogen, alkyl, haloalkyl, alkylcycloalkyl, alkylcycloalkenyl or alkoxycarbonyl, $R_5$ is hydrogen or aryl and $R_7$ is cycloalkyl, phenyl, cycloalkylalkyl or phenylalkyl;
European Patent Application No. EP 0 310 072, published April 5, 1989, discloses mimics of the Leu-Val cleavage site of angiotensinogen having the formula

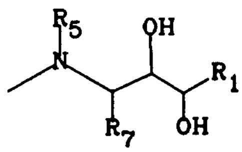

wherein $R_1$, $R_5$ and $R_7$ are as defined therein including $R_1$ is hydrogen, alkyl, haloalkyl, alkylcycloalkyl, alkylcycloalkenyl or alkoxycarbonyl, $R_5$ is hydrogen or alkyl and $R_7$ is cycloalkyl, phenyl, cycloalkylalkyl or phenylalkyl;
European Patent Application No. EP 0 310 073, published April 5, 1989, discloses mimics of the Leu-Val cleavage site of angiotensinogen having the formula

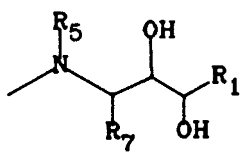

wherein $R_1$, $R_5$ and $R_7$ are as defined therein including $R_1$ is hydrogen, alkyl, haloalkyl, alkylcycloalkyl, alkylcycloalkenyl or alkoxycarbonyl, $R_5$ is hydrogen or alkyl and $R_7$ is cycloalkyl, phenyl, cycloalkylalkyl or phenylalkyl;
European Patent Application No. EP 0 313 847, published May 3, 1989, discloses mimics of the Leu-Val cleavage site of angiotensinogen having the formula

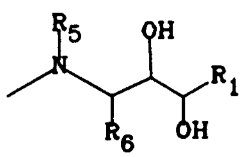

wherein $R_1$, $R_5$ and $R_6$ are as defined therein including $R_1$ is hydrogen, alkyl, haloalkyl, alkylcycloalkyl, alkylcycloalkenyl or alkoxycarbonyl, $R_5$ is hydrogen or alkyl and $R_6$ is cycloalkyl, phenyl, cycloalkylalkyl or phenylalkyl;
European Patent Application No. EP 0 296 581, published December 28, 1988, discloses mimics of the Leu-Val cleavage site of angiotensinogen having the formula

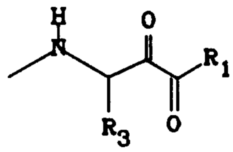

wherein $R_1$ and $R_3$ are as defined therein including $R_1$ is hydrogen, arylalkyl, aryl, (heterocycic)alkyl or heterocyclic and $R_3$ is hydrogen, alkyl, haloalkyl, arylalkyl, (heterocyclic)alkyl, hydroxyalkyl, alkoxyalkyl, aminoalkyl, mercaptoalkyl, thioalkoxyalkyl, hydroxyalkoxyalkyl, aminoalkoxyalkyl, hydroxythioalkoxyalkyl, carboxyalkyl, aminothioalkoxyalkyl, guanidinoalkyl, aminocarbonylalkyl or imidazolylalkyl;

European Patent Application No. EP 0 231 919, published August 12, 1987, discloses mimics of the Leu-Val cleavage site of angiotensinogen having the formula

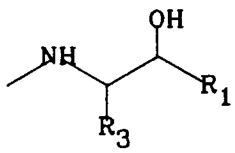

wherein $R_1$ and $R_3$ are as defined therein including $R_1$ is an N-heterocyclic ring and $R_3$ is hydrogen, alkyl, cycloalkylalkyl, haloalkyl, arylalkyl, (heterocyclic)alkyl, hydroxyalkyl, alkoxyalkyl, aminoalkyl, mercaptoalkyl, thioalkoxyalkyl, hydroxyalkoxyalkyl, aminoalkoxyalkyl, hydroxythioalkoxyalkyl, carboxyalkyl, aminothioalkoxyalkyl, guanidinoalkyl, aminocarbonylalkyl or imidazolylalkyl;

PCT Patent Application No. WO 87 05302, published May 3, 1989, discloses mimics of the Leu-Val cleavage site of angiotensinogen having the formula

$-E_{10}-F_{11}-G_{12}-H_{13}-I_{14}-Z$

wherein $E_{10}$, $F_{11}$, $G_{12}$, $H_{13}$, $I_{14}$ and Z are as defined therein including $-E_{10}-F_{11}-$ is

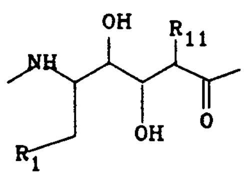

or

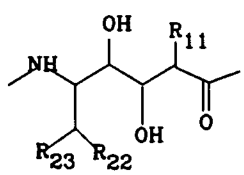

wherein $R_1$ is hydrogen, alkyl, aryl, cycloalkyl, heterocyclic, alkoxy or thioalkoxy, $R_{11}$ is hydrogen, alkyl, benzyl, cycloalkyl, hydroxyalkyl, cycloalkylalkyl, arylalkyl, (heterocyclic)alkyl, alkoxyalkyl or thioalkoxyalkyl, $R_{22}$ is hydrogen or alkyl and $R_{23}$ is hydroxyalkyl, aminoalkyl, aryl or alkyl, $G_{12}$ is absent or an amino acid residue and Z is hydroxy, substituted alkoxy, substituted amino or cyclic amino;

PCT Patent Application No. WO 87 02986, published May 21, 1987, discloses mimics of the Leu-Val cleavage site of angiotensinogen having the formula

$-E_{10}-F_{11}-G_{12}-H_{13}-I_{14}-Z$

wherein $E_{10}$, $F_{11}$, $G_{12}$, $H_{13}$, $I_{14}$ and Z are as defined therein including $-E_{10}-F_{11}-$ is

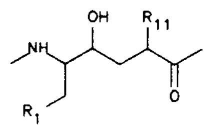 , 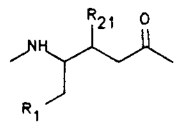 ,

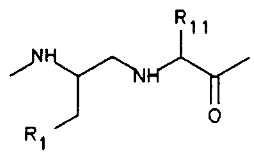 , 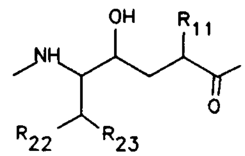 ,

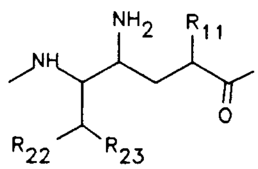 or 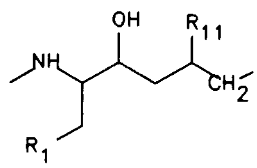

wherein $R_1$ is hydrogen, alkyl, aryl, cycloalkyl, heterocyclic, alkoxy or thioalkoxy, $R_{11}$ is hydrogen, alkyl, benzyl, cycloalkyl, hydroxyalkyl, cycloalkylalkyl, arylalkyl, (heterocyclic)alkyl, alkoxyalkyl or thioalkoxyalkyl, $R_{21}$ is hydroxy or amino, $R_{22}$ is hydrogen or alkyl and $R_{23}$ is hydroxy, amino, hydroxyalkyl, aminoalkyl, aryl or alkyl, $G_{12}$ is absent or an amino acid residue, $H_{13}$ is absent or an amino acid residue, $I_{14}$ is absent or an amino acid residue and Z is hydroxy, substituted alkoxy, substituted amino or cyclic amino;

PCT Patent Application No. WO 98 00161, published January 12, 1989, discloses mimics of the Leu-Val cleavage site of angiotensinogen having the formula

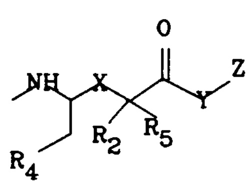

wherein $R_2$, $R_4$, $R_5$, X, Y and Z are as defined therein including $R_2$ is hydrogen or alkyl, $R_4$ is hydrogen, alkyl, cycloalkyl, aryl, heterocyclic, hydroxyalkyl or aminoalkyl, $R_5$ is hydrogen, alkyl, arylalkyl, (heterocyclic)alkyl or cycloalkyl, X is $-CH(OH)-$, $-CH(NH_2)-$, $-C(O)-$, $-CH(OH)CH(OH)-$, $-CH(OH)CH_2-$, $-CH(NH_2)CH_2-$, $-C(O)-CH_2-$, $-CH_2-NH-$, $-CH_2-O-$ or $-P(O)(A)B-$ wherein A is hydroxy or amino and B is absent, O, NH or $CH_2$, Y is absent or $-NHCH(R_5)C(O)-$ and Z is hydroxy, substituted alkoxy, substituted amino or N-heterocyclic; PCT Patent Application No. WO 88 07053, published September 22, 1988, discloses mimics of the Leu-Val cleavage site of angiotensinogen having the formula

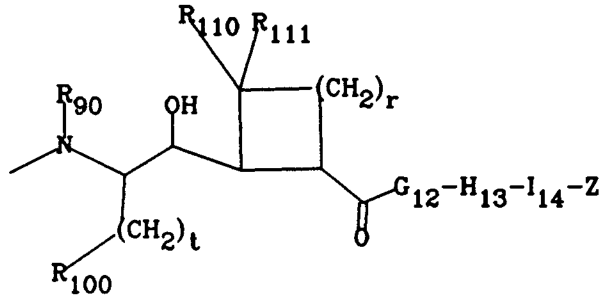

wherein r, t, $R_{90}$, $R_{100}$, $R_{110}$, $R_{111}$, $G_{12}$, $H_{13}$, $I_{14}$ and Z are as defined therein including r is 0-3, t is 0-3, $R_{90}$ is hydrogen or alkyl, $R_{100}$ is hydrogen, alkyl, aryl, cycloalkyl, heterocyclic, alkoxy or thioalkoxy, $R_{110}$ and $R_{111}$ are independently selected from hydrogen, alkyl, aryl, arylalkyl and halo, $G_{12}$ is absent, an amino acid residue or

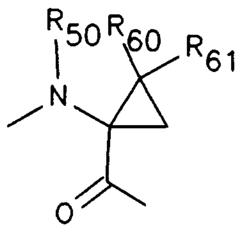

wherein $R_{50}$ is hydrogen, alkyl, arylalkyl, (heterocyclic)alkyl, cycloalkylalkyl or adamantyl, and $R_{60}$ and $R_{61}$ are independently selected from hydrogen, alkyl, aryl, arylalkyl, heterocyclic, (heterocyclic)alkyl, cycloalkyl, cycloalkylalkyl and adamantyl; or $R_{60}$ and $R_{61}$ taken together form a carbocyclic or heterocyclic spirocycle, $H_{13}$ is absent an amino acid residue or

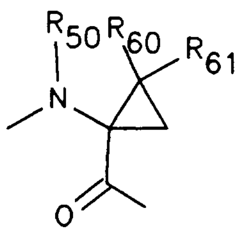

wherein $R_{50}$ is hydrogen, alkyl, arylalkyl, (heterocyclic)alkyl, cycloalkylalkyl or adamantyl, and $R_{60}$ and $R_{61}$ are independently selected from hydrogen, alkyl, aryl, arylalkyl, heterocyclic, (heterocyclic)alkyl, cycloalkyl, cycloalkylalkyl and adamantyl; or $R_{60}$ and $R_{61}$ taken together form a carbocyclic or heterocyclic spirocycle, $I_{14}$ is absent an amino acid residue or

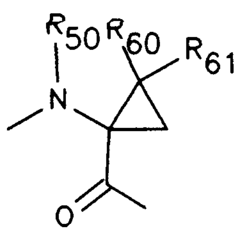

wherein $R_{50}$ is hydrogen, alkyl, arylalkyl, (heterocyclic)alkyl, cycloalkylkyl or adamantyl, and $R_{60}$ and $R_{61}$ are independently selected from hydrogen, alkyl, aryl, arylalkyl, heterocyclic, (heterocyclic), cycloalkyl, cycloalkylalkyl and adamantyl; or $R_{60}$ and $R_{61}$ taken together form a carbocyclic or heterocyclic spirocycle and Z is hydroxy, alkoxy, substituted alkoxy, amino, substituted amino or cyclic amino;
PCT Patent Application No. WO 88 02374, published April 7, 1988, discloses mimics of the Leu-Val cleavage site of angiotensinogen having the formula

a) $-E_{10}-F_{11}-C(=Y)-G_{12}-H_{13}-Z$,

b) $-E_{10}-F_{11}-W$,

c) $-E_{10}-F_{11}-G_{12}-H_{13}-W$ or

d) $-E_{10}-F_{11}-G_{121}-H_{131}-I_{14}-Z$

wherein $E_{10}$, $F_{11}$, $G_{12}$, $H_{13}$, $G_{121}$, $H_{131}$, $I_{14}$, W, Y and Z are defined therein including $-E_{10}-F_{11}-$ is

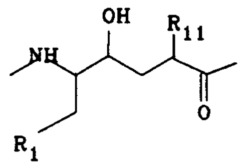 , 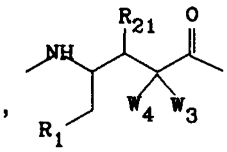 ,

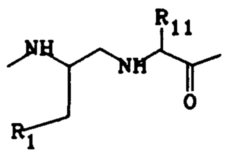 , 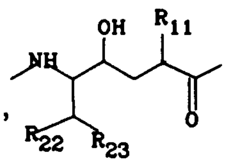 ,

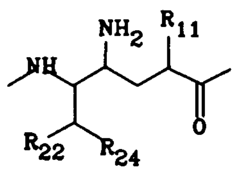 , 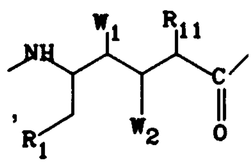 ,

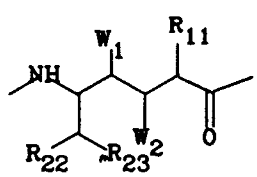 , 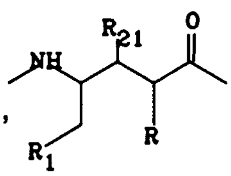 ,

or

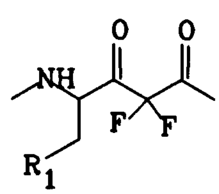

wherein R and $R_1$ are independently selected from alkyl, cycloalkyl, aryl, substituted alkyl as defined therein, alkoxy or thioalkoxy, $R_{11}$ is alkyl, cycloalkyl, aryl, substituted alkyl as defined therein, alkoxy, thioalkoxy, hydrogen, hydroxyalkyl, cycloalkylalkyl, arylalkyl, (heterocyclic)alkyl, alkoxyalkyl and thioalkoxyalkyl, $R_{22}$ is hydrogen or alkyl, $R_{23}$ is hydroxy, hydroxyalkyl, amino, aminoalkyl, aryl or alkyl, $R_{24}$ is aryl, amino, alkylamino, dialkylamino, trialkylamino, heterocyclic, hydroxy, alkoxy, alkanoyloxy, mercapto, carboxy, alkoxycarbonyl, dialkylaminoalkoxycarbonyl, aminocarbonyl, alkylaminocarbonyl, dialkylaminocarbonyl, cyclicamino, cycloalkylamino, guanidinyl, cyano, N-cyanoguanidinyl, cyanoamino, hydroxyalkylamino, di(hydroxyalkyl)amino, arylalkyl, aminoalkyl, alkylaminoalkyl, dialkylaminoalkyl, trialkylaminoalkyl, heterocyclicalkyl, hydroxyalkyl, alkoxyalkyl, alkanoyloxyalkyl, mercaptoalkyl, carboxyalkyl, alkoxycarbonylalkyl, dialkylaminoalkoxycarbonylalkyl, aminocarbonylalkyl, alkylaminocarbonylalkyl, dialkylaminocarbonylalkyl, cyclicaminoalkyl, cycloalkylaminoalkyl, guanidinylalkyl, cyanoalkyl, N-cyanoguanidinylalkyl, cyanoaminoalkyl, hydroxyalkylaminoalkyl or di(hydroxyalkyl)aminoalkyl, $W_1$ and $W_2$ are independently selected from hydroxy and amino, $W_3$ and $W_4$ are independently selected from hydrogen and fluoro, W is as defined therein, Y is O, S, NH or -N(alkyl)-, Z is as defined therein, $G_{12}$ is absent or an amino acid residue, $H_{13}$ is absent or an amino acid residue, $G_{121}$ is absent or an amino acid residue, $H_{131}$ is absent or

an amino acid residue and $I_{14}$ is absent or an amino acid residue;
PCT Patent Application No. WO 86 06379, published April 5, 1989, discloses mimics of the Leu-Val cleavage site of angiotensinogen having the formula

-E-F-G-H-Z

wherein E, F, G, H and Z are as defined therein including -E-F- is

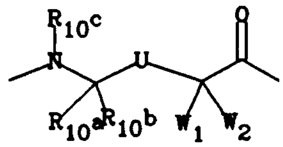

wherein $R_{10a}$ is hydrogen or alkyl, $R_{10b}$ is alkyl, cycloalkyl, cycloalkylalkyl, arylalkyl, (heteroyclic)alkyl, cycloalkenyl or cycloalkenylalkyl, $R_{10c}$ is hydrogen or alkyl, U is -C(O)-, -CH(OH)-or -CH($NH_2$)- and $W_1$ and $W_2$ are independently selected from hydrogen, fluoro, chloro and bromo, G is absent or an amino acid residue, H is absent or an amino acid residue and Z is hydroxy, thiol, amino, substituted alkoxy, substituted thioalkoxy, substituted alkylamino, Lys-OH, Lys-$NH_2$, Ser-OH or Ser-$NH_2$;
European Patent Application No. EP 0 271 862, published June 22, 1988, discloses mimics of the Leu-Val cleavage site of angiotensinogen having the formula

-Y-W-U

wherein Y, W and U are as defined therein including Y is Sta, Cysta or PhSta, W is Leu, Ile, N-MeLeu, Val or absent and U is $-NHCH_2CH(CH_3)CH_2CH_3$, $-NHCH_2Ph$, $-NHCH(CH_2OH)CH(CH_3)CH_2CH_3$, $-NHCH_2CH(OH)CH_2SCH(CH_3)_2$, $-NHCH_2CH(OH)CH_2S(O)CH(CH_3)_2$, $-NHCH_2CH(OH)CH_2S(O)_2CH(CH_3)_2$, $-NHCH_2CH_2Ph$, $-NHCH_2$(pyrid-2-yl), $-NH_2$, $-NHCH_2CH=CH_2$, -OEt, -OMe, -NH(piperidine-4-yl),

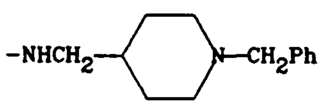 ,

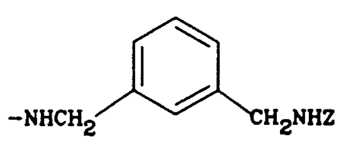 , 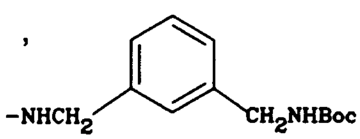 ,

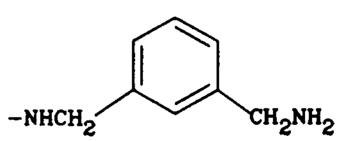 or 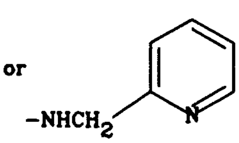

European Patent Application No. EP 0 275 480, published July 27, 1988, discloses mimics of the Leu-Val cleavage site of angiotensinogen having the formula

-W-U-V

wherein W, U and V are as defined therein including W is Sta, PhSta or Cysta, U is absent, Leu, Ile, Val, N-MeLeu or N-Melle and V is $-NHCH_2Ph$, $-NHCH_2$-cyclohexyl, -NH(piperidine-4-yl), $-NHCH_2$(pyrid-2-yl), $-NHCH_2CH(CH_3)CH_2CH_3$, -OMe, -OEt, $-NHCH(CH_2OH)CH(CH_3)CH_2CH_3$, $-NHCH_2CH_2$(morpholine-1-yl),

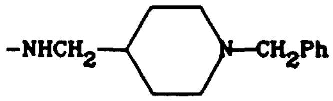

,

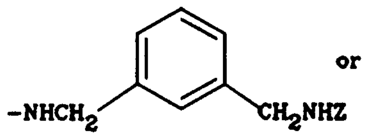

or

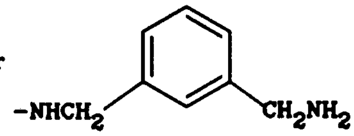

PCT Patent Application No. WO 88 03927, published June 2, 1988, discloses mimics of the Leu-Val cleavage site of angiotensinogen having the formula

$-T-(C)_n-W-(D)_n-V-(E)_n-U$

wherein T, C, W, D, V, E, U and n are as defined therein including n is 0-1, T is Sta, PhSta, Cysta, Leu, CyclohexylAla or Phe, W is absent, Leu, Gly or Ile, v is absent, Leu or Ile, C is $-CH_2NH-$, $-CH(OH)CH_2-$ or $-CH(OH)-CH=CH-C(O)-$, D is $-CH_2NH-$, E is $-CH_2NH-$ or $-CH_2N(Cbz)-$ and U is $-NHCH_2Ph$, $-NHCH_2$-cyclohexyl, $-NH_2$, -NH(piperidine-4-yl), $-NHCH_2CH(CH_3)CH_2CH_3$, -OMe, -OEt,

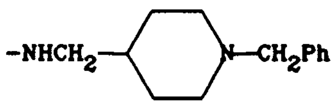

,

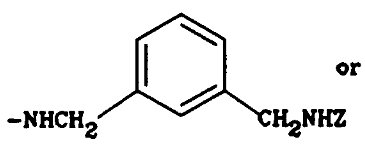

or

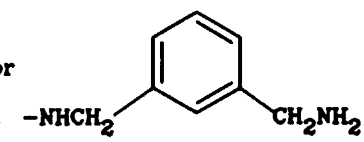

European Patent Application No. EP 0 314 060, published May 3, 1989, discloses mimics of the Leu-Val cleavage site of angiotensinogen having the formula

-W-U

wherein W and U are as defined therein including W is Sta, Cysta, PhSta, ChSta, DFKSta, DFKCys, DFKChs, ASta or ACys and U is $-NHCH_2CH_2$(morpholine-1-yl), $-NHCH_2CH(CH_2OH)-CH(CH_3)CH_2CH_3$, $-LeuNHCH_2Ph$, $-LeuNHCH_2$-cyclohexyl, -LeuNH(piperidine-4-yl), $-LeuNHCH_2$(pyrid-2-yl) or

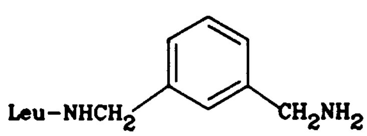

European Patent Application No. EP 0 310 918, published April 12, discloses mimics of the Leu-Val cleavage site of angiotensinogen having the formula

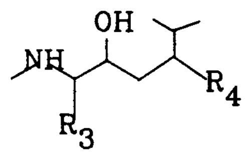

wherein $R_3$ and $R_4$ are as defined therein including $R_3$ is isobutyl, cyclohexylmethyl or benzyl and $R_4$ is phenyl, furyl, vinyl, ethyl or 1,2-dihydroxyethyl;
French Patent Application No. FR 8 700 560, published July 22, 1988, discloses mimics of the Leu-Val cleavage site of angiotensinogen having the formula

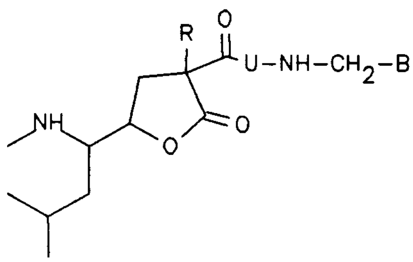

wherein R, U and B are as defined therein including R is hydrogen or hydroxyalkyl, U is Leu, Ala, Val or Ile and B is pyridyl;
European Patent Application No. EP 0 236 948, published September 16, 1987, discloses mimics of the Leu-Val cleavage site of angiotensinogen having the formula

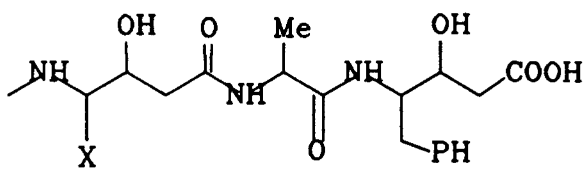

wherein X is as defined therein including X is isobutyl or benzyl;
European Patent Application No. EP 0 281 316, published September 7, 1988, discloses mimics of the Leu-Val cleavage site of angiotensinogen having the formula

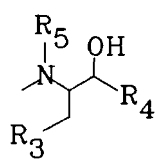

wherein $R_3$, $R_4$ and $R_5$ are as defined therein including $R_3$ is allyl, cyclohexyl or phenyl, $R_4$ is nitromethyl, alkoxycarbonyl or $-CH_2S(O)_n-R^d$ wherein n is 0-2 and $R^d$ is heterocyclic and $R_5$ is hydrogen or alkyl;
German Patent Application No. DE 38 25 242, published February 9, 1989, discloses mimics of the Leu-Val cleavage site of angiotensinogen having the formula

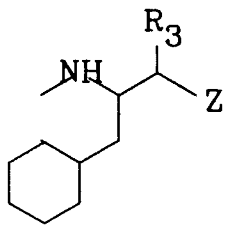

wherein $R_3$ and Z are as defined therein including $R_3$ is hydroxy or amino and Z is substituted carbonyl, substituted thiocarbonyl, substituted iminocarbonyl or unsubstituted or substituted phosphono, aminomethyl, thiomethyl, sulfinylmethyl, sulfonylmethyl or phosphonomethyl;

European Patent Application No. EP 0 275 101, published July 20, 1988, discloses mimics of the Leu-Val cleavage site of angiotensinogen having the formula

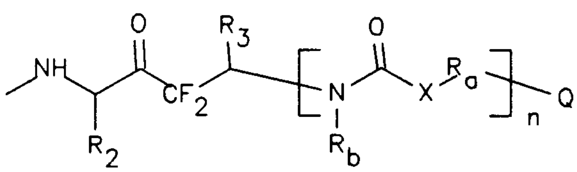

wherein $R_2$, $R_3$, $R_a$, $R_b$, n, X and Q are as defined therein including $R_2$ is an amino acid side chain, $R_3$ is hydrogen, alkyl, cyclohexyl, cyclohexylmethyl, phenyl, benzyl, 2-pyridylmethyl or an amino acid side chain, $R_a$ is an amino acid side chain, $R_b$ is hydrogen or alkyl or $R_a$ and $R_b$ taken together are $-CH_2-CH_2-$, n is 1-10, X is hydrogen, $CH_2$, alkoxy, substituted alkoxy, alkyl, phenyl, benzyl, cyclohexyl, cyclohexylmethyl or 2-pyridylmethyl and Q is hydrogen, alkyl, arylalkyl, alkoxycarbonyl or an amino acid residue;

PCT Patent Application No. WO 89 01488, published February 23, 1989, discloses mimics of the Leu-Val cleavage site of angiotensinogen having the formula

$-E_{10}-F_{11}-G_{12}-H_{13}-I_{14}-Z$

wherein $E_{10}$, $F_{11}$, $G_{12}$, $H_{13}$, $I_{14}$ and Z are as defined therein including $-E_{10}-F_{11}-$ is

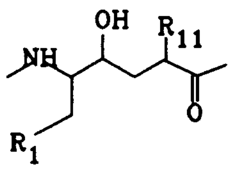, 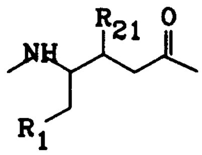 ,

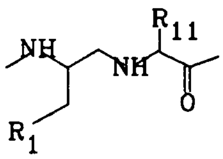, 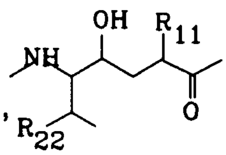 ,

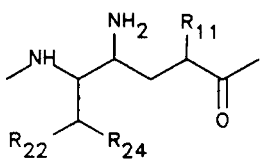, 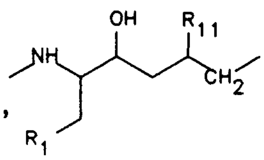 ,

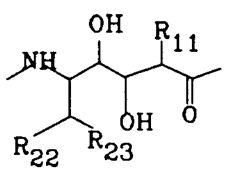, 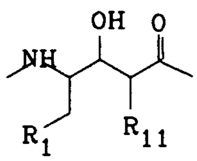 ,

or 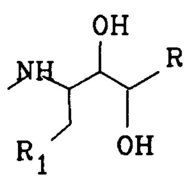

wherein $R_1$ is hydrogen, alkyl, aryl, cycloalkyl, heterocyclic, alkoxy or thioalkoxy, $R_{11}$ is hydrogen, alkyl, benzyl, cycloalkyl, hydroxyalkyl, cycloalkylalkyl, arylalkyl, (heterocyclic)alkyl, alkoxyalkyl or thioalkoxyalkyl, $R_{21}$ is hydroxy or amino, $R_{22}$ is hydrogen or alkyl and $R_{23}$ is hydroxy, amino, hydroxyalkyl, aminoalkyl, aryl or alkyl, $R_{24}$ is $R_1$ hydroxy, amino, hydroxyalyl or aminoalkyl, $G_{12}$ is absent or an amino acid residue, $H_{13}$ is absent or an amino acid residue, $I_{14}$ is absent or an amino acid residue and Z is hydroxy, substituted alkoxy, substituted amino or cyclic amino;

European Patent Application No. EP 0 275 101, published July 20, 1988, discloses mimics of the Leu-Val cleavage site of angiotensinogen having the formula

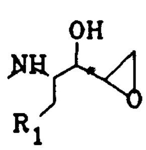, 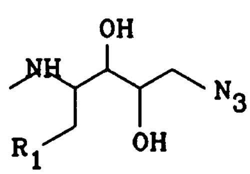 ,

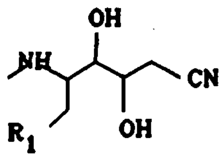 or 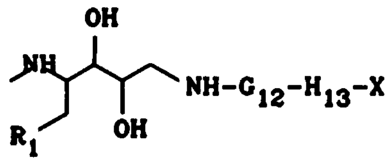

wherein $R_1$, $G_{12}$, $H_{13}$ and X are as defined therein including $R_1$ is hydrogen, alkyl, aryl, cycloalkyl, heterocyclic, alkoxy or thioalkoxy, $G_{12}$ is absent an amino acid residue or an amino acid residue wherein the alpha-amino group has been replaced by O, $H_{13}$ is absent, an amino acid residue or an amino acid residue wherein the alpha-amino group has been replaced by O and X is hydrogen, alkyl or substituted alkyl as defined therein; European Patent Application No. EP 0 312 291, published April 19, 1989, discloses mimics of the Leu-Val cleavage site of angiotensinogen having the formula

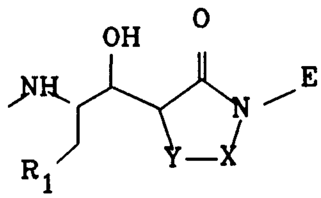

wherein $R_1$, Y, X and E are as defined therein including $R_1$ is hydrogen, alkyl, aryl, cycloalkyl, 1,3-dithiolan-2-yl or 1,3-dithian-2-yl, X is $-CH_2-C(R_{13})(R_{14})-$ wherein $R_{13}$ and $R_{14}$ are independently selected from hydrogen, alkyl, alkenyl, carboxy, aminocarbonyl, substituted aminocarbonyl, substituted alkyl, alkanoyloxy, substituted aminocarbonyloxy, substituted carbonylamino, substituted aminocarbonylamino, substituted sulfinyl, substituted sulfonyl, substituted sulfide, amino, alkylamino, dialkylamino or heterocyclic, Y is $CH_2$, O, S, SO or $SO_2$ or X and Y taken together is $-(CH_2)_4-$ and E is hydrogen, aryl, heterocyclic, alkyl, cycloalkyl or substituted alkyl;

European Patent Application No. EP 0 312 283, published April 19, 1989, discloses mimics of the Leu-Val cleavage site of angiotensinogen having the formula

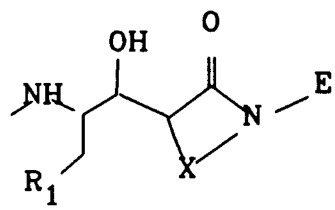

wherein $R_1$, X and E are as defined therein including $R_1$ is hydrogen, alkyl, aryl, cycloalkyl, 1,3-dithiolan-2-yl or 1,3-dithian-2-yl, X is $-CH_2-C(R_{13})(R_{14})-$ wherein $R_{13}$ and $R_{14}$ are independently selected from hydrogen, alkyl, alkenyl, carboxy, aminocarbonyl, substituted aminocarbonyl, substituted alkyl, alkanoyl, substituted aminocarbonyloxy, substituted carbonylamino, substituted aminocarbonylamino, substituted sulfinyl, substituted sulfonyl, substituted sulfide, amino, alkylamino, dialkylamino or heterocyclic and E is hydrogen, aryl, heterocyclic, alkyl, cycloalkyl or substituted alkyl;

European Patent Application No. EP 0 312 158, published April 19, 1989, discloses mimics of the Leu-Val cleavage site of angiotensinogen having the formula

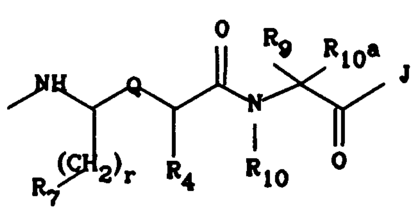

wherein r, $R_7$, $R_4$, $R_{10}$, $R_9$, $R_{10a}$, Q and J are as defined therein including r is 1-4, $R_7$ is alkyl, aryl or cycloalkyl, $R_4$ is hydrogen, alkyl, alkenyl, cycloalkyl, aryl or substituted alkyl, $R_{10}$ and $R_{10a}$ are independently selected from hydrogen and alkyl, $R_9$ is $-(CH_2)_s-NR_{11}R_{12}$ wherein s is 1-2 and $R_{11}$ and $R_{12}$ are independently selected from hydrogen, heterocyclic, aryl cycloalkyl, alkyl, arylalkyl, (heterocyclic)alkyl, aminoalkyl, hydroxyalkyl, alkylaminoalkyl, dialkylaminoalkyl, carboxy, alkyl substituted by $-SO_3H$, aminocarbonylalkyl, alkylaminocarbonylalkyl or dialkylaminocarbonylalkyl, Q is -CH(OH)-, $-CH(N(R_8))-$, $-CH(OH)CH_2-$ or
$-CH(N(R_8))CH_2-$ wherein $R_8$ is hydrogen, alkyl, formyl, alkanoyl, aroyl, alkoxycarbonyl, aryloxycarbonyl or arylalkoxycarbonyl and J is substituted alkylamino or substituted alkoxy; European Patent Application No. EP 0 312 157, published April 19, 1989, discloses mimics of the Leu-Val cleavage site of angiotensinogen having the formula

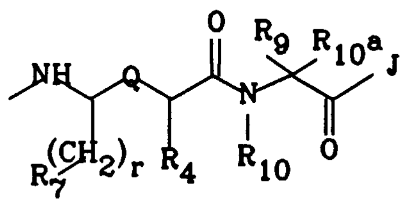

wherein r, $R_7$, $R_4$, $R_{10}$, $R_9$, $R_{10a}$, Q and J are as defined therein including r is 1-4, $R_7$ is alkyl, aryl or cycloalkyl, $R_4$ is hydrogen, alkyl, alkenyl, cycloalkyl, aryl or substituted alkyl, $R_{10}$ and $R_{10a}$ are independently selected from hydrogen and alkyl, $R_9$ is $-(CH_2)_s-NR_{11}R_{12}$ wherein s is 1-2 and $R_{11}$ and $R_{12}$ are independently selected from hydrogen, heterocyclic, aryl, cycloalkyl, alkyl, arylalkyl, (heterocyclic)alkyl, aminoalkyl, hydroxyalkyl, alkylaminoalkyl, dialkylaminoalkyl, carboxy, alkyl substituted by $-SO_3H$, aminocarbonylalkyl, alkylaminocarbonylalkyl or dialkylaminocarbonylalkyl, Q is -CH(OH)-, $CH(N(R_8))-$, $-CH(OH)CH_2-$ or $-CH(N(R_8))CH_2-$ wherein $R_8$ is hydrogen, alkyl, formyl, alkanoyl, aroyl, alkoxycarbonyl, aryloxycarbonyl or arylalkoxycarbonyl and J is substituted alkylamino, substituted alkoxy, heterocyclic, heterocyclicamino or substituted guanidino; European Patent Application No. EP 0 314 239, published May 3, 1989, discloses mimics of the Leu-Val cleavage site of angiotensinogen having the formula

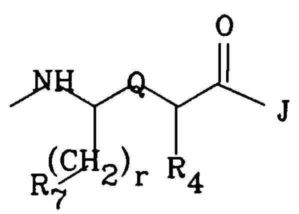

wherein r, $R_7$, $R_4$, Q and J are as defined therein including r is 1-4, $R_7$ is alkyl, aryl or cycloalkyl, $R_4$ is hydrogen, alkyl, alkenyl, cycloalkyl, aryl or substituted alkyl, Q is -CH(OH)-, $-CH(N(R_8))-$, $-CH(OH)CH_2-$ or $-CH(N(R_8))CH_2-$ wherein $R_8$ is hydrogen, alkyl, formyl, alkanoyl, aroyl, alkoxycarbonyl, aryloxycarbonyl or araylalkoxycarbonyl and J is amino, hydroxy, substituted alkylamino or substituted alkoxy;
South African Patent Application No. 866642, published February 24, 1987, discloses mimics of the Leu-Val cleavage site of angiotensinogen having the formula

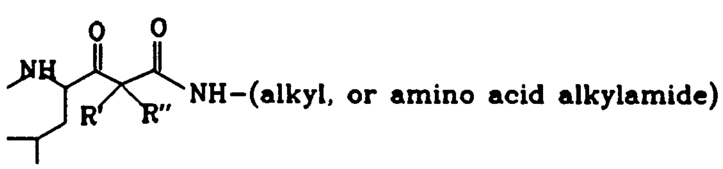

wherein R and R" are as defined therein including R' is fluoro and R" is hydrogen or fluoro; European Patent Application No. EP 0 273 696, published July 6, 1988, discloses mimics of the Lern-Val cleavage site of angiotensinogen having the formula

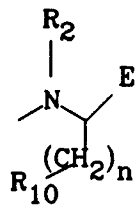

wherein n, $R_2$, $R_{10}$ and E are as defined therein including n is 0-5, $R_2$ is hydrogen or alkyl, $R_{10}$ is alkyl, cycloalkyl, cycloalkylalkyl, arylalkyl, (heterocyclic)alkyl, alkoxyalkyl, thioalkoxyalkyl, hydroxyalkyl or aminoalkyl and E is -CH(W)-G wherein W is hydroxy, amino, alkanoyloxy or alkanoyloxyalkyloxy and G is -Q-C(O)-T-U-V wherein Q is a bond or $-CH(R_{13})-$ wherein $R_{13}$ is hydrogen, aryl, alkyl, cycloalkyl or substituted alkyl, T and U are independently absent or selected from an amino acid residue and V is hydroxy, substituted alkoxy, amino or substituted amino;

European Patent Application No. EP 0 278 158, published August 17, 1988, discloses mimics of the Leu-Val cleavage site of angiotensinogen having the formula

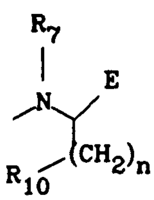

wherein n, $R_7$, $R_{10}$ and E are as defined therein including n is 0-3, $R_7$ is alkyl or substituted alkyl, $R_{10}$ is alkyl, cycloalkyl, cycloalkylalkyl, arylalkyl, (heterocyclic)alkyl, alkoxyalkyl, thioalkoxyalkyl, hydroxyalkyl or aminoalkyl and E is -CH(W)-G wherein W is hydroxy, amino, alkanoyloxy or alkanoyloxyalkyloxy and G is -Q-C(O)-T-U-V wherein Q is a bond or $-CH(R_{13})-$wherein $R_{13}$ is hydrogen, aryl, alkyl, cycloalkyl or substituted alkyl, T and U are independently absent or selected from an amino acid residue and V is hydroxy, substituted alkoxy, amino or substituted amino;

German Patent Application No. DE 37 21 855, published September 22, 1988, discloses mimics of the Leu-Val cleavage site of angiotensinogen having the formula

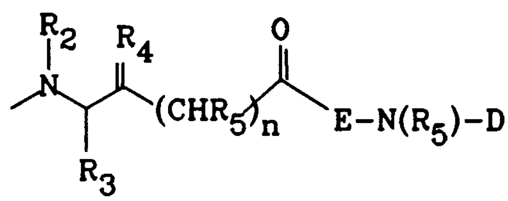

wherein n, $R_2$, $R_3$, $R_4$, $R_5$, $R_6$, E and D are as defined therein including n is 1-2, $R_2$ is hydrogen or alkyl, $R_3$ is hydrogen, alkyl, aryl, arylalkyl, (heterocyclic)alkyl, cycloalkyl, alkoxy or cycloalkylalkyl, $R_4$ is (H, OH), (H, $NH_2$) or O, $R_5$ is hydrogen or alkyl, $R_6$ is hydrogen or alkyl, E is 0-2 amino acid residues and D is $-CH_2CHOHCH_2OH$, substituted sulfonyl, substituted sulfonylalkyl, substituted carbonyl, substituted phosphonyl, phenyl, phenylalkyl, furyl, furylalkyl, thienyl, thienylalkyl, pyridyl, pyridylalkyl or other (heterocyclic)alkyl; European Patent Application No. EP 0 309 841, published April 5, 1989, discloses mimics of the Leu-Val cleavage site of angiotensinogen having the formula

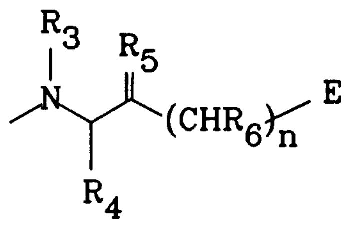

wherein n, $R_3$, $R_4$, $R_5$, $R_6$ and E are as defined therein including n is 1-2, $R_3$ is hydrogen or alkyl, $R_4$ is hydrogen, alkyl, aryl, arylalkyl, heterocyclic, (heterocyclic)alkyl, cycloalkyl, alkoxy or cycloalkylalkyl, $R_5$ is (H, OH), (H, $NH_2$) or O, $R_6$ is hydrogen, alkyl or alkenyl and E is $-SR_7$, $-SOR_7$, $-SO_2R_7$, $-SO_2OR_7$ or $-SO_2NR_7R_8$ wherein $R_7$ and $R_8$ are independently selected from $R_4$; European Patent Application No. EP 0 292 800, published November 30, 1988, discloses mimics of the Leu-Val cleavage site of angiotensinogen having the formula

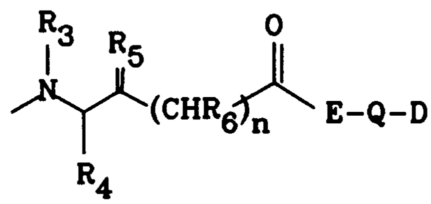

wherein n, $R_3$ $R_4$, $R_5$, $R_6$, E, Q and Y are as defined therein including n is 1-2, $R_3$ is hydrogen or alkyl, $R_4$ is hydrogen, alkyl, aryl, arylalkyl, heterocyclic, (heterocyclic)alkyl, cycloalkyl, cycloalkylalkyl or alkoxy, $R_5$ is (H, OH), (H, $NH_2$) or O, $R_6$ is hydrogen or alkyl, E is 0-2 amino acid residues, Q is O or NH and Y is H or substituted alkyl;

European Patent Application No. EP 0 249 096, published December 16, 1987, discloses mimics of the Leu-Val cleavage site of angiotensinogen having the formula

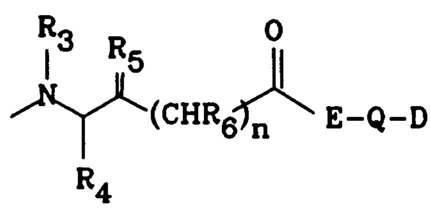

wherein n, $R_3$, $R_4$, $R_5$, $R_6$, E, Q and Y are as defined therein including n is 1-2, $R_3$ is hydrogen or alkyl, $R_4$ is hydrogen, alkyl, aryl, arylalkyl, heterocyclic, (heterocyclic)alkyl, cycloalkyl, cycloalkylalkyl or alkoxy, $R_5$ is (H, $OR_{12}$), (H, $NR_{12}R_{13}$), or O wherein $R_{12}$ and $R_{13}$ are independently selected from hydrogen and alkyl, $R_6$ is hydrogen or alkyl, E is 0-2 amino acid residues, Q is O or NH and Y is H or substituted alkyl;

European Patent Application No. EP 0 264 795, published April 27, 1988, discloses mimics of the Leu-Val cleavage site of angiotensinogen having the formula

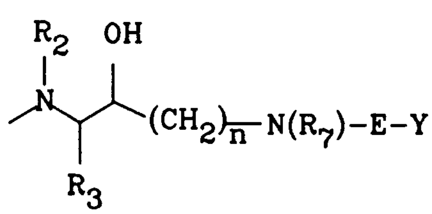

wherein n, $R_2$, $R_3$, $R_4$, E and Y are as defined therein including n is 1-2, $R_2$ is hydrogen or alkyl, $R_3$ is hydrogen, alkyl, aryl, arylalkyl, heterocyclic, (heterocyclic)alkyl, cycloalkyl, cycloalkylalkyl or alkoxy, $R_4$ is hydrogen or alkyl, E is -C(O)NH-, -C(S)NH-, -C(O)O-, $-SO_2-$, $-SO_2NH-$ or -PO(OA)O- wherein A is hydrogen or alkyl and Y is carboxy, carboxyalkyl, substituted carboxyalkyl, alkoxycarbonyl, alkoxycarbonylalkyl, substituted alkoxycarbonylalkyl, aminocarbonyl, substituted aminocarbonyl, aminocarbonylalkyl, substituted aminocarbonylalkyl, hydrogen, alkyl, aryl, arylalkyl, cycloalkyl or cycloalkylalkyl; or E-Y is pyrrolidinocarbonyl, piperidinocarbonyl, morpholinocarbonyl, pyrrolidinosulfonyl, piperidinosulfonyl or morpholinosulfonyl.

The term "substituted methylene group" as used herein refers to:

(I) $-CHR_{13}R_{14}$ wherein

1) $R_{13}$ is
- i) hydrogen or
- ii) hydroxy

and

2) $R_{14}$ is
- i) hydrogen,
- ii) loweralkyl,
- iii) hydroxy,
- iv) hydroxyalkyl,
- v) alkoxy,
- vi) alkoxyalkyl,
- vii) azido,
- viii) azidoalkyl,
- ix) amino,
- x) (N-protected)amino,
- xi) aminoalkyl,
- xii) (N-protected)aminoalkyl,
- xiii) alkylamino,
- xiv) (N-protected)(alkyl)amino,
- xv) alkylaminoalkyl,
- xvi) (N-protected)(alkyl)aminoalkyl
- xvii) dialkylamino,
- xviii) dialkylaminoalkyl,
- xix) carboxyalkyl,
- xx) thioalkoxy,
- xxi) thioalkoxyalkyl,
- xxii) alkylsulfonyl,
- xxiii) alkylsulfonylalkyl,
- xxiv) thioaryloxy,
- xxv) thioaryloxyalkyl,
- xxvi) arylsulfonyl,
- xxvii) arylsulfonylalkyl,
- xxviii) (unsubstituted heterocyclic)alkyl or
- xxix) (substituted heterocyclic)alkyl such that when $R_{13}$ is hydroxy then $R_{14}$ is not hydroxy, alkoxy, azido, amino, alkylamino, dialkylamino, (N-protected)amino, (N-protected)(alkyl)amino, thioalkoxy, alkylsulfonyl or arylsulfonyl, and such that when $R_{13}$ is hydrogen then $R_{14}$ is not hydrogen or loweralkyl;

(II) $-C(=CH_2)C(O)NHA_{15}$,

(III) $-C(OH)(R_{16})C(O)NHR_{15}$ or

(IV) $-CH(R_{16})C(O)NHA_{15}$ wherein

1) $R_{15}$ is
- i) loweralkyl,
- ii) hydroxyalkyl,
- iii) alkoxyalkyl,
- iv) aminoalkyl,
- v) alkylaminoalkyl,
- v) dialkylaminoalkyl,
- vi) dialkylaminoalkyl,
- vii) heterocyclic,

viii) aryl,
ix) (heterocyclic)alkyl or
x) arylalkyl and
2) $R_{16}$ is
i) hydrogen,
ii) loweralkyl,
iii) hydroxyalkyl,
iv) haloalkyl or v) azidoalkyl;

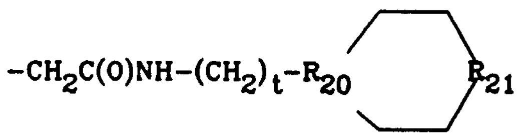

wherein
1) t is 0 to 3,
2) $R_{20}$ is
i) $CH_2$ or
ii) N and
3) $R_{21}$ is
i) NH,
ii) O,
iii) S or
iv) $SO_2$, such that when t is 0 then $R_{20}$ is $CH_2$ and when t is 1 to 3 then $R_{20}$ is N,
(VI) $-CH_2CH(R_{22})C(O)NHA_{23}$ wherein
1) $R_{22}$ is
i) loweralkyl or
ii) cycloalkylalkyl
and
2) $R_{23}$ is
i) loweralkyl,
ii) hydroxyalkyl,
iii) alkoxyalkyl,
iv) aminoalkyl,
v) alkylaminoalkyl,
vi) dialkylaminoalkyl,
vii) heterocyclic,
viii) aryl,
ix) (heterocyclic)alkyl,
x) arylalkyl or
xi)

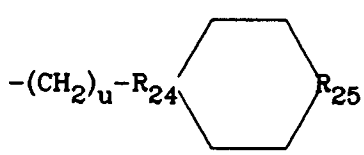

wherein
a) u is 0 to 3,
b) $R_{24}$ is $CH_2$ or N and
c) $R_{25}$ is NH, O, S or $SO_2$,
such that when u is 0 then $R_{24}$ is $CH_2$ and when u is 1 to 3 then $R_{24}$ is N;

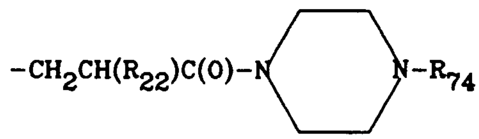

wherein
1) $R_{22}$ is as defined above and
2) $R_{74}$ is
i) hydrogen,
ii) loweralkyl,
iii) an N-protecting group or
iv) $R_{75}$-C(O)- wherein $R_{75}$ is aminoalkyl or (N-protected)aminoalkyl;

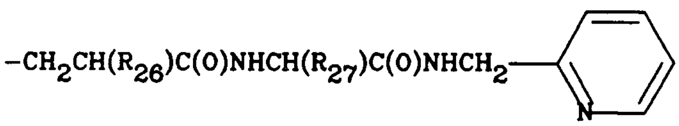

wherein
1) $R_{26}$ is
i) loweralkyl or
ii) cycloalkylalkyl and
2) $R_{27}$ is
i) loweralkyl or
ii) cycloalkylalkyl;

(IX) $-CH_2CH(R_{81})NHC(O)R_{82}$ or $-CH_2CH(R_{81})NHS(O)_2R_{82}$ wherein
1) $R_{81}$ is
i) loweralkyl or
ii) cycloalkylalkyl and
2) $R_{82}$ is
i) loweralkyl,
ii) alkoxy,
iii) alkylamino,
iv) dialkylamino,
v) -OR* wherein R* is aminoalkyl, alkylaminoalkyl, dialkylaminoalkyl or (heterocyclic)alkyl or
vi)

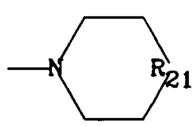

as defined above;

(X) $-CH_2NH(CO)R_{82}$ or $-CH_2NHS(O)_2R_{82}$ wherein $R_{82}$ is as defined above, or

(X) $-CF_2CH(OH)R_{83}$ wherein $R_{83}$ is loweralkyl, loweralkenyl, cycloalkyl, cycloalkenyl, cycloalkylalkyl, cycloalkenylalkyl, aryl, arylalkyl, heterocyclic or (heterocyclic)alkyl, or physiologically compatible salts thereof.

The chiral centers of the compounds of the invention may have either the "R", "S" or "R,S" configuration. The terms "R" and "S" configuration are as defined by IUPAC 1974 Recommendations for Section E, Fundamental Stereochemistry, Pure Appl. Chem. (1976) 45, 13-30.

An aliphatic residue is preferentially an alkyl residue, which may be saturated or unsaturated. The same is true for residues derived therefrom, e.g. alkoxy, alkylthio, alkylamino, dialkylamino, alkanoyl or aralkyl.

An alicyclic ring may be for instance cycloalkyl or a residue derived therefrom. A $(C_3-C_8)$-cycloalkyl residue

is preferentially cyclopropyl, cyclobutyl, cyclopentyl, cyclohexyl, cycloheptyl or cyclooctyl. If one of these cycles contains more than one substituent, they may be positioned cis or trans.

A ($C_6$-$C_{12}$)-aralkyl residue is for instance phenyl, naphthyl or biphenyl; phenyl is preferred. The same is true for residues derived therefrom, e.g. aryloxy, aroyl, aralkyl and aralkyloxy. Araliphatic residues are for instance aralkyl-residues. These are preferentially an unsubstiuted or substituted ($C_6$-$C_{12}$)-aryl residue connected to a ($C_1$-$C_6$)-alkyl residue.

A heteroaromatic residue is a group containing a sixnumbered aromatic ring derived from benzene, in which one or more CH-groups are displaced by nitrogen or a five-numbered aromatic ring derived from benzene, in which 2 CH-groups are displaced by S, NH or N and in which further CH-groups may be displaced by N. Their heteroaromatic ring may be mono-or -bicyclic. In the latter case it may be anellated with a benzene ring or with a ring as defined above. The same is true for residues derived therefrom, e.g. hetaryl, hetaroyl and hetaryloxy.

A heterocyclic residue in the sense of this definition is for instance a residue from the following group: pyrrolyl, furyl, thienyl, imidazolyl, pyrazolyl, oxazolyl, thiazolyl, pyridyl, pyrazinyl, pyrimidoyl, indolyl, quinolinyl, isoquinolinyl, quinoxalinyl, $\beta$-carbolinyl, benzofuranyl, benzoxazolyl, benzthiazolyl.

Halogen is fluorine, chlorine, bromine or iodine.

(h + k) is preferentially $\geqq$2.

(i + e) is preferentially $\geqq$1.

(q + r) is preferentially $\geqq$1.

A substituted residue in the sense of the above definition is a residue, which is optionally substituted by one, two or three residues from the series ($C_1$-$C_6$)-alkyl, ($C_1$-$C_6$)-alkoxy, hydroxy, halogen, ($C_1$-$C_6$)-alkanoyl, $CF_3$, amino, ($C_1$-$C_6$)-alkylamino, Di-($C_1$-$C_6$)-alkylamino, carbamoyl, ($C_1$-$C_6$)-alkoxycarbonyl or sulfamoyl.

Salts of the compounds of this invention are preferentially pharmaceutically compatible and non-toxic salts. Such salts are formed for instance by compounds of formula I containing acidic groups with cations of main groups I and II of the periodic table, e.g. Na, K, Mg or Ca or with physiologically compatible amines, e.g. triethylamine or tris-(2-hydroethyl)-amino. Compounds of formula I containing basic groups form salts with inorganic acids like hydrochloric acid, sulfonic acid or phosphoric acid or with organic acid, like acetic acid, citric acid, benzoic acid, maleic acid, fumaric acid, tartaric acid or p-toluenesulfonic acid.

Preferred are compounds of formula I, in which

$R^A$ and $R^B$ are the same or different and are hydrogen, ($C_1$-$C_4$)-alkyl, ($C_4$-$C_6$)-cycloalkyl, ($C_4$-$C_6$)-cycloalkyl-($C_1$-$C_4$)-alkyl, ($C_6$-$C_{12}$)-aryl, which may be substituted by one or two identical or different residues of the group methyl, ethyl, methoxy, ethoxy, halogen or amino,
partially hydrogenated ($C_6$-$C_{12}$)-aryl,
($C_6$-$C_{12}$)-aryl-($C_1$-$C_4$)-alkyl, which may be substituted in the aryl part as defined above, ($C_1$-$C_6$)-alkanoyl,
($C_6$-$C_{12}$)-aryl-($C_1$-$C_4$)-alkanoyl, which may be substituted in the aryl part as defined above,
($C_4$-$C_{10}$)-hetaryl-($C_1$-$C_4$)-alkanoyl,
($C_1$-$C_4$)-alkoxy-($C_1$-$C_4$)alkanoyl,
($C_1$-$C_4$)-alkoxycarbonyl-($C_1$-$C_6$)-alkanoyl,
($C_6$-$C_{12}$)-aryloxycarbonyl-($C_1$-$C_6$)-alkanoyl, which may be substituted in the aryl part as defined above,
($C_6$-$C_{12}$)-aryl-($C_1$-$C_4$)-alkoxycarbonyl, which may be substituted in the aryl part as defined above or in which

| | |
|---|---|
| $R^A$ and $R^B$ | form together with the N-atom bearing them a piperidino-, pyrrolidino-, morpholino-, piperazine or thiomorpholino-ring; or in which |
| $R^A$ | is defined as above and |
| $R^B$ | is amino, methylamino, ethylamino, di-($C_1$-$C_2$-)alkylamino, hydroxy, methoxy, ethoxy, methylsulfonyl, ethylsulfonyl, phenylsulfonyl or carbamoyl; |
| $R^F$ | is defined as for $R^A$ and $R^B$ above; |
| $R^G$ | is hydrogen, ($C_1$-$C_3$)-alkyl, ($C_4$-$C_6$)-cycloalkyl, ($C_4$-$C_6$)-cycloalkyl-($C_1$-$C_2$)-alkyl, ($C_6$-$C_{12}$)-aryl or ($C_6$-$C_{12}$)-aryl-($C_1$-$C_2$)-alkyl; |

$R^C$, $R^D$ and $R^E$ are same or different and are hydrogen or ($C_1$-$C_3$)-alkyl or $R^D$ and $R^E$ form an ethylene bridge;

| | |
|---|---|
| h | is 0, 1 or 2; |
| i | is 0, 1 or 2; |
| k | is 1,2 or 3; |
| l | is 1, 2 or 3; |
| X | is -CO- or -$SO_2$-; |
| Y | is -$(CH_2)_q$-$(CR^HR^L)_r$ or -O-, wherein |
| | q is 0, 1 or 2, |
| | r is 0, 1 or 2, |

$R^H$ and $R^L$ are the same or different and are hydrogen or $(C_1-C_3)$-alkyl;

Z is $-CH_2-$ or absent and

all other residues are defined as above.

Especially preferred are compounds of formula I, in which

$R^A$, $R^B$ and $R^F$ are the same or different and are hydrogen, $(C_1-C_4)$-alkyl, $(C_1-C_4)$-alkanoyl, e.g. acetyl, $(C_6-C_{12}$-aryl-$(C_1-C_4)$-alkyl, e.g. benzoyl; $(C_7-C_{13})$-aroyl, e.g. benzyl, $(C_4-C_{10})$-hetaroyl, e.g. nicotinoyl, $(C_1-C_7)$-alkoxycarbonyl, e.g. t.-butoxycarbonyl, or benzyloxycarbonyl;

$R^G$ is hydrogen or methyl;

$R^C$, $R^D$ and $R^E$ are the same or different and are hydrogen, methyl or ethyl or $R^D$ and $R^E$ form an ethylene bridge;

h is 0, 1 or 2;

i is 0, 1 or 2;

k is 1, 2 or 3;

l is 1, 2 or 3;

X is -CO- or $-SO_2-$;

Y is $-CH_2-$ or -O-;

Z is $-CH_2-$ or absent;

$R_2$ is cyclohexylmethyl, benzyl, 1- or 2-naphthylmethyl, 2-,3- or 4-thienylmethyl, p-methoxybenzyl, p-fluorobenzyl, most preferentially benzyl and in which all other residues are defined as above.

The invention also comprises a procedure for preparation of compounds of formula I, which consists of reacting a fragment with a C-terminal carboxy group or its reactive derivative with another fragment with a free N-terminal amino group, optionally removing temporarily introduced protecting groups and optionally transferring the compound so obtained into its physiologically compatible salt. Fragments of a compound of formula I with a C-terminal carboxyl group have the formulae (IVa) and (IVb), respectively:

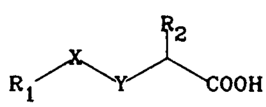
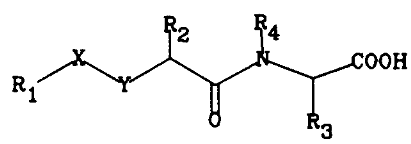

(IVa) (IVb)

Fragments of a compound of formula I with an N-terminal amino group have the formulae Va and Vb respectively:

$$R_4\text{-HN-CH}(R_3)\text{-CO-T} \quad \text{(Va)} \qquad\qquad \text{H-T} \quad \text{(Vb)}$$

The condensation of the components is executed only on the combination IVa with Va and IVb with Vb, respectively. Methods suitable for the formation of amide bonds are described for instance in Houben-Weyl, Methoden der organischen Chemie, Vol. 15/2; Bodanszky et al., Peptide synthesis, 2nd ed. (Wiley & Sons, New York 1976) or Gross, Meienhofer, The Peptides. Analysis, Synthesis, Biology (Academic press, New York 1979). Preferentially, the following methods are used:

Active ester coupling using N-hydroxysuccinimide or 1-hydroxybenzotriazole as ester components, coupling with a carbodiimide like dicyclohexylcarbodiimide or with propanphosphonic anhydride or the mixed-anhydride coupling using pivaloylchloride (M. Zaoval, Coll. Chem. Comann 1962, 27 1273). The reaction is run in an inert solvent or solvent mixture between -20 ° C and the boiling point of the solvent used.

The preparation of optically active components Vb is known and described in the references cited above.

The preparation of carboxy-protected succinic acid derivatives (a subgroup of IVa) is accomplished according to J.J. Plattner et al. (J. Med. Chem. 31, 2277 (1988)) and D.A. Eveans et al. (J. Amer. Chem. Soc. 104, 1737 (1982)).

The preparation of carbamoyl substituted α-hydroxypropionic acid (another subgroup of IVa) derivatives is accomplished by reaction of enantiomerically pure or racemic α-hydroxyproionic acid derivatives and the

described N-containing ring systems $R_1$-H using reactive carbonic acid derivatives, e.g. phosgene, diphosgene, triphosgene, carbonyldiimidazol or di-(1-benzotriazolyl)-carbonate with addition of suitable bases, e.g. triethylamine, pyridine or ethyl diisopropylamine in an inert solvent.
Aminosulfonyl derivatives (another subgroup of IVa) are prepared by condensation of the appropriate amines with ω-Chlorosulfonyl-alkylcarboxylic acid derivatives with the addition of a suitable base. As an example, chlorosulfonylpropionic acid derivatives are obtained from the addition of suitable S-containing nucleophiles to substituted acrylic acid derivatives and ensuing oxidation of the S-atom.
The ancillary operations necessary for the preparation of compounds of formula I, e.g. introduction and removal of protecting groups are know from the literature. They are, for instance, described in T.W. Grenne "Protective Groups in Organic Synthesis". (Wiley & Sons, New York 1981). Salts of compounds of the formula I having salt-forming groups are prepared in a manner which is known per se, for example by reacting a compound of the formula I having a basic group with a stoichiometric amount of a suitable acid, or reacting compounds of the formula I having an acid group with a stoichiometric amount of a suitable base. Stereoisomer mixtures, in particular diastereomer mixtures, which are obtained, if appropriate, during the synthesis of compounds of the formula I, can be resolved in a manner which is known per se by fractional crystallization or by chromatography.

The compounds of the formula I according to the invention have enzyme-inhibiting properties, and in particular they inhibit aspartyl proteases, such as renin.

Renin is secreted into the blood circulation from the juxtaglomerular cells of the kidney as a consequence of various stimuli (volume depletion, sodium deficiency, β-receptor stimulation). In the blood circulation it splits off the decapeptide angiotensin I from the angiotensinogen discharged by the liver. This decapeptide is converted by angiotensin converting enzyme (ACE) into angiotensin II. Angiotensin II plays an essential role in blood pressure regulation, since it directly increases the blood pressure by vasoconstriction. It additionally stimulates the secretion of aldosterone from the adrenals and in this manner increases the extracellular fluid volume via inhibition of sodium excretion, which in turn contributes towards increasing the blood pressure. Inhibitors of the enzymatic activity of renin have the effect of a reduced formation of angiotensin I, the consequence of which is a reduced formation of angiotensin II. The reduction in the concentration of this active peptide hormone is the direct cause of the antihypertensive action of renin inhibitors.

The activity of renin inhibitors can be investigated by in vitro tests. In these, the reduction in the formation of angiotensin I is measured in various systems (human plasma and purified human renin).

**1. Test principle**

For example, human plasma which contains both renin and angiotensinogen is incubated at 37 °C with the compound to be tested. During this incubation, angiotensin I is liberated from angiotensinogen by the action of renin and can then be measured using a commercially available radioimmunoassay. This angiotensin liberation is inhibited by renin inhibitors.

**2. Isolation of the plasma**

The blood is obtained from volunteers (about 0.5 l per person; Bluko sampler from ASID Bonz and Sohn, Unterschleißheim) and collected in partly evacuated bottles, while cooling with ice. Coagulation is prevented by addition of EDTA (final concentration 10 mM). After centrifugation (Rotor HS 4 (Sorvall)), 3,500 revolutions per minute, 0-4 °C, 15 minutes; repeated if necessary), the plasma is carefully pipetted off and frozen at -30 °C in suitable portions. Only plasmas having a sufficiently high renin activity are used for the test. Plasmas having a low renin activity are activated (prorenin → renin) by a low temperature treatment (-4 °C, 3 days).

**3. Test procedure**

Angiotensin I is determined with a Renin-Maia® kit (Serono Diagnostics S.A., Coinsins, Switzerland). The plasma is incubated in accordance with the instructions given with the kit:

Incubation batch: 1000 µl of plasma (thawed at 0-4 °C)
100 µl of phosphate buffer (pH 7.4, addition of $10^{-4}$ M ramiprilat)
10 µl PMSF solution
10 µl of 0.1 % of ®Genapol PFIC
12 µl of dimethyl sulfoxide or test preparation

The test preparations are in general dissolved as a $10^{-2}$ M solution in 100 % pure dimethyl sulfoxide (DMSO) and the solutions are diluted accordingly with water; the incubation mixture contains not more than 1 % of DMSO.

The mixtures are mixed in ice and placed in a waterbath (37 ° C) for 1 hour for incubation. A total of 6 samples (in each case 100 µl) are removed from an additional mixture without an inhibitor and without further incubation in order to determine the starting angiotensin I content of the plasma used.

The concentrations of the test preparations are chosen so that approximately the range of 10-90 % enzyme inhibition is covered (at least five concentrations). At the end of the incubation period, three 100 µl samples from each mixture are frozen on dry ice in pre-cooled Eppendorf vessels and kept at about -25 ° C for the angiotensin I determination (mean value of three individual samples).

**4. Angiotensin I radioimmunoassay (RIA)**

The instructions for using the RIA kit (Renin-Maia® kit, Serono Diagnostics S.A., Coinsins, Switzerland) are followed exactly.

The calibration plot includes the range from 0.2 to 25.0 ng of angiotensin I per ml. The basal angiotensin I content of the plasma is subtracted from all the measured values. The plasma renin activity (PRA) is stated as ng of Ang I/ml x hour. PRA values in the presence of the test substances are related to a mixture without inhibitor (= 100 %) and stated as % residual activity. The $IC_{50}$ value is read off from the plot of % residual activity against the concentration (M) of the test preparation (logarithmic scale).

The compounds of the general formula I described in the present invention exhibit inhibitory actions at concentrations of about $10^{-5}$ to $10^{-10}$ mo/l in the in vitro test.

Renin inhibitors cause a reduction in blood pressure in animals with salt depletion. Since human renin differs from the renin of other species, primates, such as, for example, Rhesus monkeys, are used for in vivo testing of renin inhibitors.

**1. Test principle**

Primate renin and human renin are largely homologous in their sequence. An endogenous secretion of renin is stimulated by intravenous injection of furosemide. The test compounds are then administered and their effect on blood pressure and heart rate is measured.

**2. Test procedure**

6 Rhesus monkeys were pretreated orally with 10 mg/kg x day of furosemide on 6 successive days. On the 7th day, a further 10 mg/kg of furosemide were administered intravenously about 30 minutes before the start of the experiment. Anaesthesia was then induced with 20 mg/kg of ketamine intramuscularly and continued with 40 mg/kg of pentobarbitone intravenously. A side arm of the femoral artery was exposed and a cannula inserted for blood pressure measurement by means of a blood pressure transducer (P 23 ID). Blood samples for determination of the plasma renin activity were removed via a Braunüle in the saphenous vein.

The compounds of the present invention are effective here in a dose range of about 0.1-5 mg/kg intravenously, and in the dose range from about 0.5-50 mg/kg on intraduodenal administration by a gastroscope.

The compounds of the general formula I described in the present invention can be used as anti-hypertensives and for the treatment of cardiac insufficiency.

HIV-protease splits itself auto-catalytically from the gag-pol-polypeptide and subsequently hydrolyses the precursor protein P 55 into the core antigens P 17, P 24 and P 14. It is an essential enzyme, the inhibition of which interrupts the life cycle of the HIV-virus and impedes its proliferation.
Biological test have shown, that the compounds of the present invention inhibit viral enzymes, especially HIV-protease. This property qualifies the compounds of the present invention as agents for therapy and prophylaxis of viral infections, especially of HIV infectins. The compounds of formula I show $IC_{50}$ values in the range of $10^{-4}$-$10^{-9}$ M in vitro-enzyme assays used.
The invention furthermore relates to the use of compounds of the formula I for the preparation of pharmaceuticals for antihypertensive therapy treatment of congestive cardiac insufficiency and treatment and prophylaxis of HIV-infections.

Pharmaceutical preparation contain an active amount of the active compound of the formula I together with an inorganic or organic pharmaceutically usable excipient.

They can be used intranasally, intravenously, subcutaneously, perorally or intraduodenally. The dosage of the active compound depends on the warm-blooded species, the body weight, the age and the mode of administration.

The pharmaceutical preparations of the present invention are prepared in dissolving, mixing, granulating or tablet-coating processes which are known per se.

For the oral use forms, the active compounds are mixed with the additives customary for these, such as excipients, stabilizers or inert diluents, and the mixture is brought by customary methods into suitable presentation forms, such as tablets, coated tablets, two-piece capsules, aqueous, alcoholic or oily suspensions or aqueous, alcoholic or oily solutions. Inert excipients which can be used are, for example, gum arabic, magnesia, magnesium carbonate, potassium phosphate, lactose, glucose, magnesium stearyl fumarate or starch, in particular maize starch. The formulation here can be in the form either of dry granules or of moist granules. Examples of possible oily excipients or solvents are vegetable or animal oils, such as sunflower oil and cod-liver oil.

For subcutaneous or intravenous administration, the active compounds or physiologically tolerated salts thereof are dissolved, suspended or emulsified if desired with the substances customary for this purpose, such as solubilizing agents, emulsifiers or other auxiliaries. Possible solvents are, for example: water, physiological saline solutions or alcohols, for example ethanol, propanediol or glycerol, and in addition also sugar solutions, such as glucose solutions or mannitol solutions, or a mixture of the various solvents mentioned.

List of abbreviations used:

| | |
|---|---|
| BOC | tert.butoxycarbonyl |
| DCC | dicyclohexylcarbodiimide |
| DMF | dimethylformamide |
| EA | ethylacetate |
| FAB | fast atom bombardment |
| HBTU | O-benzotriazol-1-yl-N,N,N',N'-tetramethyl-uronium |
| His | L-histidine |
| HOBT | 1-hydroxybenzotriazol |
| NEM | N-ethyl-morpholine |
| RT | room temperature |
| TFA | trifluoro acetic acid |

**Example 1**

N-[N-(3-(4-Amino-1-piperidinyl-carbonyl)-2(R)-benzyl-propionyl)-L-histidine]-(2S,3R,4S)-1-cyclohexyl-3,4-dihydroxy-6-methyl-2-heptylamide-acetate

0.3 mmol of N-[N-(3-(4-BOC-amino-1-piperidinyl-carbonyl)-2(R)-benzyl-propionyl-L-histidine]-(2S,3R,4S)-1-cyclohexyl-3,4-dihydroxy-6-methyl-2-heptylamide are stirred at RT with 1 ml of TFA and 1 ml of $CH_2Cl_2$ for 2 hrs. After evaporation to dryness, the residue is dissolved in 3 ml of $H_2O$ and adjusted to pH 6 with ion exchange resin Amberlite® IRA 93 (acetate form). After filtration, the product is isolated by lyophilization. 0.25 mmoles of light amber powder are obtained.
MS (FAB): 653 (M + H); 659 (M + Li)

a) N-[N-(3-(4-BOC-amino-1-piperidinyl-carbonyl)-2-(R)-benzyl-propionyl-L-histidine]-(2S,3R,4S)-1-cyclohexyl-3,4-dihydroxy-6-methyl-2-heptylamide

0.4 mmoles of H-His-(2S,3R,4S)-1-cyclohexyl-3,4-dihydroxy-6-methyl-2-heptylamide (known from EP-A-332 008), 0.4 mmoles of 3-(4-BOC-amino-1-piperidinyl-carbonyl)2-(R)-benzyl-propionic acid, 0.4 mmoles 1-hydroxybenzothiazole and 0.4 mmoles of dicyclohexylcarbodiimide are dissolved in 5 ml of dry DMF. The pH is adjusted to 8.5 with N-ethyl morpholine. After stirring at RT for 24 hrs, the precipitate is filtered and the filtrate is diluted with EA. The organic layer is washed 1 x with sat. $NaHCO_3$- solution, 1 x with sat. NaCl-solution, dried over $MgSO_4$ and evaporated. Column chromatography of the crude product gave rise to the title compound as an amorphous powder.
MS (FAB): 663 (M + H); 660 (M + Li)

According to the procedures described in example 1 the following compounds were obtained using the appropriate starting materials.

**Example 2**

3-(4-Amino-1-piperidinyl-carbonyl)-2(R)-benzyl-propionyl-L-histidine amide of (2'S,1'R,5S)-3-Ethyl-5-(1'-hydroxy-2'-amino-3'-cyclohexylpropyloxazolidine-2-one-acetate

Obtained from the H-His-amide of (2'S,1'R,5S)-3-ethyl-5-(1'-hydroxy-2'-amino-3'-cyclohexylpropyl-oxazolidine-2-one (known from EP-A-307837); amorphous powder.
MS (FAB): 684 (M + H); 690 (M + Li)

**Example 3**

3-(4-Amino-1-piperidinyl-carbonyl)-2(R)-benzyl-propionyl-L-histidine amide of (2'S,1'R,5S)-3-methoxy-5-(1'-hydroxy-2'-amino-3'-cyclohexylpropyl)-oxazolidine-2-one-Acetate

Obtained from the H-His-amide of (2'S,1'R,5S)-3-methoxy-5-(1'-hydroxy-2'-amino-3'-cyclohexylpropyl-oxazolidine-2-one (known from EP-A-307837); amorphous powder.
MS (FAB): 686 (M + H); 692 (M + Li)

**Example 4**

3-(4-Amino-1-piperidinyl-carbonyl)-2(R)-benzyl-propionyl-L-histidine amide of (5S,1'R,2'S)-5-(Amino-3-cyclohexyl-1-hydroxy)-3,3-dimethyl-dihydrofura-2(3H)-one-Acetate

Obtained from the H-His-amide of (5S,1'R,2'S)-5-(2-amino-3-cyclohexyl-1-hydroxy)-3,3-dimethyl-dihydrofura-2(3H)-one (known from EP-A 307 837); amorphous powder.
MS (FAB): 682 (M + H); 688 (M + Li)

**Example 5**

3-(4-Amino-1-piperidinyl-carbonyl)-2-(R)-benzyl-propionyl-L-histidine amide of (2S,3R,4S)-1-cyclohexyl-2-amino-3,4-dihydroxy-5-morpholino-pentane diacetate

Obtained from the H-His-amide of (2S,3R,4S)-1-cyclohexyl-2-amino-3,4-dihydroxy-5-morpholino-pentane (know from EP-A 309 766); amorphous powder.
MS (FAB): 699 (M + H); 705 (M + Li)

**Example 6**

3-(4-Amino-1-piperidinyl-carbonyl)-2(R)-benzyl-propionyl-L-histidine-amide of (2S,3R,4S)-2-amino-1-cyclohexyl-3,4-dihydroxy-5-(N'-isopropyl-ureido)-pentane acetate

Obtained from the H-His-amide of (2S,3R,4S)-2-amino-1-cyclohexyl-3,4-dihydroxy-5-(N'-isopropyl-ureido)-pentane (known from the WO 88/04664); amorphous powder.
MS (FAB): 714 (M + H); 720 (M + Li)

**Example 7**

3-(4-Amino-1-piperidinyl-carbonyl)-2(R)-benzyl-propionyl-L-histidine amide of (2S,3R,4S,6RS)-2-amino-1-cyclohexyl-6-methyl-3,4,7-trihydroxy-heptane-acetate

Obtained from the H-His-amide of (2S,3R,4S,6RS)-2-amino-1-cyclohexyl-6-methyl-3,4,7-trihydroxy-heptane (known from EP-A 307 837); amorphous powder.
MS (FAB): 673 (M + H); 679 (M + Li)

**Example 8**

3-(4-Amino-1-piperidinyl-carbonyl)-2(R)-benzyl-propionyl-L-histidine amide of (2S,3R,5S)-2-amino-1-cyclohexyl-5-isopropyl-6-propylsulfonyl-amino-hexane-acetate

Obtained from the H-His-amide of (2S,3R,5S)-2-amino-1-cyclohexyl-5-isopropyl-6-propylsulfonylamino-hexane (known from EP-A 339 483); amorphous powder.
MS (FAB): 776 (M + H); 782 (M + Li)

**Example 9**

3-(4-Amino-1-piperidinyl-carbonyl)-2(R)-benzyl-propionyl-L-histidine amide of (2S,4S,1'R,2'S)-2-(2'-amino-3'-cyclohexyl-1'-hydroxyl)-4-methyltetrahydrofuran-acetate

Obtained from the H-His-amide of (2S,4S,1'R,2'S)-2-(2'-amino-3'-cyclohexyl-1'hydroxy)-4-methyl-tetrahydrofuran (known from EP-A 307 837), amorphous powder.
MS (FAB): 655 (M + H); 661 (M + Li)

**Example 10**

3-(4-Amino-1-piperidinyl-carbonyl)-2(R)-benzyl-propionyl-L-histidinyl amide of (2S,4S,5S)-5-amino-6-cyclohexyl-4-hydroxy-2-isopropyl-hexanoic acid-n-butylamide-acetate

Obtained from the H-His-amide of (2S,4S,5S)-5-amino-6-cyclohexyl-4-hydroxy-2-isopropyl-hexanoic acid-n-butylamide (known from US 4,613,676); amorphous powder.
MS (FAB): 726 (M + H); 732 (M + Li)

**Example 11**

2(S)-(4-Amino-1-piperidinyl-carbonyl-oxy)-3-phenyl-propionyl-L-histidine amide of (2S,3R,4S)-2-amino-1-cyclohexyl-3,4-dihydroxy-6-methylheptane-acetate

The title compound was synthesized from the resultant compound from Example 12 following the procedure described in Example 1.
MS (FAB): 655 (M + H); 677 (M + Na)

a) 2(S)-(4-(tert.-Butyloxycarbonyl)amino-1-piperidinyl-carbonyl-oxy)-3-phenyl-propionyl-L-histidine amide of (2S,3R,4S)-2-amino-1-cyclohexyl-3,4-dihydroxy-6-methylheptane

0.15 mmol of BOC-L-histidine amide of (2S,3R,4S)-2-Amino-1-cyclohexyl-3,4-dihydroxy-6-methylheptane (known from the European Patent Application No. 332008) was dissolved in 1 ml of dry $CH_2Cl_2$ and cooled to 0 °C under Ar atmosphere. This solution was treated with 1 ml of trifluoroacetic acid for 3 h at room temperature. After concentration under reduced pressure and drying under high vacuum the residue was dissolved in 30 ml of ethyl acetate and washed with 1N $NaHCO_3$ and brine. After drying ($Na_2SO_4$) and evaporation the resultant residue was dissolved in 2 ml of dry acetonitrile. To this solution 0.15 mmol of 2-(S)-(4-amino-1-piperdinyl-carbonyl-oxy)-3-phenyl-propionic acid, 0.15 mmol HOBt, 0.22 mmol NEM and 0.15 mmol HBTU were added. The reaction solution was stirred for 2 h at room temperature, than treated with 5 ml brine and extracted with three 10 ml portions of ethyl acetate. The combined organic phases were washed with saturated $NaHCO_3$ and brine, dried over $Na_2SO_4$ and evaporated. Purification by silica gel chromatography afforded the desired product.
MS (FAB): 755 (M + H); 777 (M + Na)

According to the procedures described in Example 11 the following compounds were synthesized using appropriate starting materials.

**Example 12**

2(S)-(4-Amino-1-piperidinyl-carbonyl-oxy)-3-phenyl-propionyl-L-histidine amide of (2'S,1'R,5S)-3-ethyl-5-(1'-hydroxy-2'-amino-3'-cyclohexyl)-propyl-oxazolidine-2-one-acetate

This compound was obtained from BOC-L-histidine amide of (2'S,1'R,5S)-3-ethyl-5(1'-hydroxy-2'-amino-3'-cyclohexyl)-propyl-oxazolidine-2-one (known from EP-A 307 837) as an amorphous powder.
MS (FAB): 682 (M+H), 688 (M+Li)

**Example 13**

2(S)-(4-Amino-1-piperidinyl-carbonyl-oxy)-3-phenyl-propionyl-L-histidine amide of (2'S,1'R,5S)-3-methoxy-5-(1'-hydroxy-2'-amino-3'-cyclohexyl)-propyl-oxazolidine-2-one-acetate

Obtained from BOC-L-histidine amide of (2'S,1'R,5S)-3-methoxy-5-(1'-hydroxy-2'-amino-3'-cyclohexylpropyl-oxazolidine-2-one (known from EP-A 307 837); amorphous powder.
MS (FAB): 684 (M+H); 690 (M+Li)

**Example 14**

2(S)-(4-Amino-1-piperidinyl-carbonyl-oxy)-3-phenyl-propionyl-L-histidine amide of (5S,1'R,2'S)-5-(2-amino-3-cyclohexyl-1-hydroxy)-3,3-dimethyl-dihydrofura-2(3H)-one-acetate

Obtained from BOC-L-histidine amide of (5S,1'R,2'S)-5-(2-amino-3-cyclohexyl-1-hydroxy)-3,3-dimethyl-dihydrofura-2(3H)one (known from EP-A 307 837) as amorphous powder.
MS (FAB): 681 (M+H); 703 (M+Na)

**Example 15**

2(S)-(4-Amino-1-piperidinyl-carbonyl-oxy)-3-phenyl-propionyl-L-histidine amide of (2S,3R,4S)-2-amino-1-cyclohexyl-3,4-dihydroxy-5-morpholino-pentane-diacetate

Obtained from BOC-L-histidine amide of (2S,3R,4S)-2-amino-1-cyclohexyl-3,4-dihydroxy-5-morpholino-pentane (known from EP-A 309 766).
MS (FAB): 698 (M+H); 720 (M+Na)

**Example 16**

2(S)-(4-Amino-1-piperidinyl-carbonyl-oxy)-3-phenyl-propionyl-L-histidine amide of (2S,3R,4S)-2-amino-1-cyclohexyl-3,4-dihydroxy-5-(N'-isopropyl-ureido)-pentane-acetate

Obtained from BOC-L-histidine amide of (2S,3R,4S)-2-amino-1-cyclohexyl-3,4-dihydroxy-5-(N'-isopropyl-ureido)-pentane (known from WO 88/04664); amorphous powder.
MS (FAB): 713 (M+H); 719 (M+Li)

**Example 17**

2-(S)-(4-Amino-1-piperidinyl-carbonyl-oxy)-3-phenyl-propionyl-L-histidine amide of (2S,3R,4S,6RS)-2-amino-1-cyclohexyl-6-methyl-3,4,7-trihydroxy-heptane-acetate

Obtained from BOC-L-histidine amide of (2S,3R,4S,6RS)-2-amino-1-cyclohexyl-methyl-3,4,7-trihydroxy-heptane (known from EP-A 307 837); amorphous powder.
MS (FAB): 671 (M+H); 677 (M+Li)

**Example 18**

2(S)-(4-Amino-1-piperidinyl-carbonyl-oxy)-3-phenyl-propionyl-L-histidine amide of (2S,3R,5S)-2-amino-1-cyclohexyl-5-isopropyl-6-propylsulfonyl-amino-hexane-acetate

Obtained from BOC-L-histidine amide of (2S,3R,5S)-2-amino-1-cyclohexyl-5-isopropyl-6-propylsulfonylamino-hexane (known from EP-A 339 483); amorphous powder.
MS (FAB): 774 (M+H), 796 (M+Na).

**Example 19**

2(S)-(4-Amino-1-piperidinyl-carbonyl-oxy)-3-phenyl-propionyl-L-histidine amide of (2S,4S,1'R,2'S)-2-(2'-amino-3'-cyclohexyl-1'-hydroxy)-4-methyltetrahydrofuran-acetate

Obtained from BOC-L-histidine amide of (2S,4S,1'R,2'S)-2-(2'-amino-3'-cyclohexyl-1'-hydroxy)-4-methyltetrahydrofuran (known from EP-A 307 837); amorphous powder.
MS (FAB): 653 (M+H); 659 (M+Li)

**Example 20**

2(S)-(4-Amino-1-piperidinyl-carbonyl-oxy)-3-phenyl-propionyl-L-histidine amide of (2S,4S,5S)-5-Amino-6-cyclohexyl-4-hydroxy-2-isopropylhexanoic acid-n-butylamide-acetate

Obtained from BOC-L-histidine amide of (2S,4S,5S)-5-amino-cyclohexyl-4-hydroxy-2-isopropyl-hexanoic acid-n-butylamide (known from US 4,613,676); amorphous powder.
MS (FAB): 724 (M+H); 746 (M+Na)

**Example 21**

3-(4-Amino-1-piperidinyl-carbonyl)-(R)-benzyl-propionyl-L-histidine amide of 3-(4-Morpholinyl)-propyl-5-(S)-amino-6-cyclohexyl-4(S)-hydroxy-2(S)-isopropylhexanamide-acetate

Obtained from 3-(4-morpholinyl)-propyl-5(S)-amino-6-cyclohexyl-4(S)-hydroxy-2(S)-isopropylhexanamide (known from EP-A 364 804); amorphous powder.
MS (FAB) 807 (M+H); 813 (M+Li)

**Example 22**

3-(4-Amino-1-piperidinyl-carbonyl)-2(R)-benzyl-propionyl-L-histidine amide of 2(S)-amino-1-cyclohexyl-5(S)-[(2-(2-pyridyl)ethoxy-carbonyl]-amino-3(S)-hydroxy-6-methylheptane-acetate

Obtained from 2(S)-amino-1-cyclohexyl-5(S)-[(2-(2-pyridyl)ethoxy-carbonyl]amino-3(S)-hydroxy-6-methylheptane (known from EP-A 364,804); amorphous powder.
MS (FAB) 801 (M+H); 807 (M+Li)

**Example 23**

3-(4-Amino-1-piperidinyl-carbonyl)-2(R)-(1-naphthylmethyl)-propionyl-L-histidine amide of (2S,3R,4S)-1-cyclohexyl-2-amino-3,4-dihydroxy-6-methylheptane-acetate

Obtained from the H-His-amide of (2S,3R,4S)-1-cyclohexyl-2-amino-3,4-dihydroxy-6-methylheptane (known from EP-A 332 008); amorphous powder.
MS (FAB): 703 (M+H); 709 (M+Li)

**Example 24**

3-(4-Amino-1-piperidinyl-carbonyl)-2(R)-(1-naphthylmethyl)-propionyl-L-histidine amide of (2S,4S,1'R,2'S)-2-(2'-amino-3'cyclohexyl-1'-hydroxy)-4-methyltetrahydrofuran-acetate

Obtained from the H-His-amide of (2S,4S,1'R,2'S)-2-(2'-amino-3'-cyclohexyl-1'-hydroxy)-4-methyltetrahydrofuran (known from EP-A 300 837); amorphous powder.
MS (FAB): 705 (M + H); 711 (M + Li) **Example 25**

3-(4-Amino-1-piperidinyl-sulfonyl)-2(R)-benzyl-propionyl-L-histidine amide of (2S,3R,4S)-1-cyclohexyl-2-amino-3,4-dihydroxy-6-methylheptane-acetate

Obtained from the H-His-amide of (2S,3R,4S)-1-cyclohexyl-2-amino-3,4-dihydroxy-6-methylheptane (known from EP-A 332 008); amorphous powder.
MS (FAB): 689 (M + H); 695 (M + Li)

**Example 26**

3-(4-Amino-1-piperidinyl-sulfonyl)-2(R)-benzyl-propionyl-L-histidine amide of (2'S,1'R,5S)-3-ethyl-5-(1'-hydroxy-2'-amino-3'-cyclohexyl)-propyl-oxazolidine-2-one-acetate

Obtained from the H-His-amide of (2'S,1'R,5S)-3-ethyl-5-(1'-hydroxy-2'-amino-3'-cyclohexyl)propyl-oxazolidin-2-one (known from EPA 307 837); amorphous powder.
MS (FAB): 720 (M + H); 726 (M + Li)

**Example 27**

3-(4-Aminomethyl-1-piperidinyl-carbonyl)-2(R)-benzyl-propionyl-L-histidine amide of (2S,4S,1'R,2'S)-2-(2'-amino-3'-cyclohexyl-1'-hydroxy)-4-methyl-tetrahydrofuran-acetate

Obtained from the H-His-amide of (2S,4S,1'R,2'S)-2-(2'-amino-3'-cyclohexyl-1'-hydroxy)-4-methyltetrahydrofuran (known from EP-A-307 837); amorphous powder.
MS (FAB): 669 (M + H); 675 (M + Li)

## Claims

**1.** A compound of the formula I

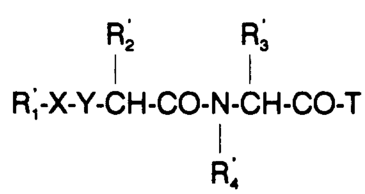

(I)

in which the residues have the following meaning:

$R'_1$ a residue of formula II or III

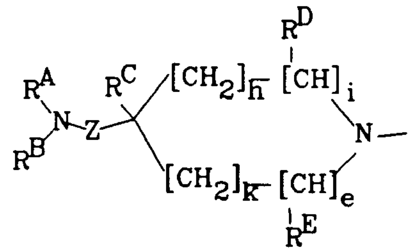

(II)

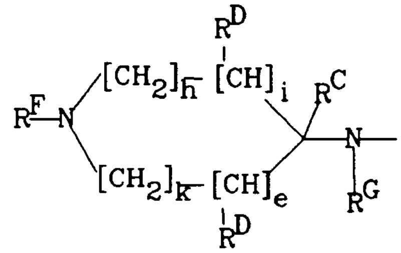

(III)

in which

| | |
|---|---|
| $R^A$ and $R^B$ | may be the same or different and are hydrogen, unsubstituted $(C_1\text{-}C_{21})$-alkyl, unsubstituted or substituted $(C_3\text{-}C_{20})$-Cycloalkyl, unsubstituted or substituted $(C_4\text{-}C_{20})$-cycloalkyl-alkyl, substituted or unsubstituted $(C_6\text{-}C_{12})$-Aryl, unsubstituted or substituted $(C_6\text{-}C_{12})$-Aryl-$(C_1\text{-}C_{20})$-alkyl, unsubstituted or substituted $(C_5\text{-}C_{12})$-hetaryl-$(C_1\text{-}C_8)$-alkyl, or, if not covered by the definitions above are unsubstituted or substituted $(C_1\text{-}C_{18})$-alkanoyl), unsubstituted or substituted $(C_3\text{-}C_8)$-cycloalkyl-$(C_1\text{-}C_8)$-alkanoyl, substituted or unsubstituted $(C_7\text{-}C_{13})$-Aroyl, unsubstituted or substituted $(C_5\text{-}C_{13})$-heteroaroyl, unsubstituted or substituted $(C_6\text{-}C_{12})$-aryl-$(C_1\text{-}C_{18})$-alkanoyl, unsubstituted or substituted $(C_5\text{-}C_{13})$-hetaryl-$(C_1\text{-}C_{18})$-alkanoyl or unsubstituted or substituted $(C_6\text{-}C_{12})$-aryl-$(C_1\text{-}C_4)$-alkoxycarbonyl<br>or<br>$R^A$ and $R^B$ form together with the nitrogen atom bearing them a 4-8-numbered heterocyclic ring which may be unsaturated or saturated and which may contain another heteroatom from the group N, O or S;<br>or<br>$R^A$ is defined as above and $R^B$ is<br>unsubstituted or substituted $(C_1\text{-}C_4)$-alkylamino,<br>unsubstituted or substituted Di-$(C_1\text{-}C_4)$-alkylamino, hydroxy,<br>unsubstituted or substituted $(C_1\text{-}C_4)$-alkoxy,<br>unsubstituted or substituted $(C_1\text{-}C_4)$-alkylsulfonyl,<br>unsubstituted or substituted $(C_6\text{-}C_{12})$-arylsulfonyl or carbamidoyl; |
| $R^F$ | has the meaning as defined above for $R^A$ and $R^B$ and may be the same or different; |
| $R^G$ | is hydrogen, $(C_1\text{-}C_8)$-alkyl, $(C_3\text{-}C_8)$-cycloalkyl, $(C_3\text{-}C_8)$-cycloalkyl-$(C_1\text{-}C_4)$-alkyl, unsubstituted or substituted $(C_6\text{-}C_{12})$-aryl, unsubstituted or substituted $(C_7\text{-}C_{13}$-aralkyl; |
| $R^C$, $R^D$ and $R^E$ | may be the same or different and are hydrogen, $(C_1\text{-}C_6)$-alkyl or |
| $R^D$ and $R^E$ | together form a $(C_1\text{-}C_4)$-alkylene bridge; |
| h | is 0, 1, 2 or 3; |
| i | is 0, 1, 2 or 3; |
| k | is 1, 2, 3 or 4; |
| l | is 1, 2, 3 or 4; |
| X | is -CO-, -CS-, $SO_2$ - or -SO-; |
| Y | is $(CH_2)_q$-$(CR^HR^L)_r$-, -O- or -S-, in which<br>q = 0, 1, 2 or 3;<br>r = 0, 1 or 2;<br>$R^H$ and $R^L$ are the same or different and hydrogen or $(C_1\text{-}C_6)$-alkyl; |

Z is absent or a straight chain or branched ($C_1$-$C_6$)-alkylene-residue;

$R'_2$ is hydrogen, ($C_1$-$C_{10}$)-alkyl; ($C_6$-$C_{12}$)-aryl; ($C_6$-$C_{12}$)-aryl-($C_1$-$C_4$)-alkyl; ($C_3$-$C_8$)-cycloalkyl; ($C_3$-$C_8$)-cycloalkyl-($C_1$-$C_4$)-alkyl, hetaryl or hetaryl-($C_1$-$C_4$)-alkyl, aryl and hetaryl being optionally substituted by one, two or three residues from the group halogen, hydroxy, ($C_1$-$C_4$)-alkoxy, $CF_3$, ($C_1$-$C_4$)-alkyl;

$R'_3$ is the side chain of a natural or unnatural amino acid, preferably from the group phenylalanine, histidine, tyrosine, tryptophan, methionine, leucine, isoleucine, asparagine, aspartic acid, $\beta$-2-furyl alanine, $\beta$-3-furyl alanine, lysine, ornithine, valine, alanine, 2,4-diaminobutyric acid, arginine, 4-chlorophenyl alanine, methionine sulfone, methionine sulfoxide, 2-pyridylalanine, 3-pyridylalanine, cyclohexylalanine, cyclohexylglycine, O-tert.-butyl tyrosine, phenylglycine, 1-naphthylalanine, 2-naphthylalanine, 4-nitrophenylalanine, norvaline, $\beta$-2-benzo[b]thienylalanine, $\beta$-3-benzo-[b]thienylalanine, 2-fluorophenylalanine, 3-fluorophenylalanine, 4-fluorophenylalanine, norleucine, cysteine, S-methylcysteine, 1,2,3,4-tetrahydroisoquinoline-3-carboxylic acid, homophenylalanine, DOPA, O-dimethyl-DOPA, N-methyl-histidine, 2-amino-4-(2-thienyl)-butyric acid, 2-amino-4-(3-thienyl)-butyric acid, 3-(2-thienyl)-serine, 2- or 4-thiazolyl alanine, 1,3-thioxolane-2-yl-alanine, 3-(N-pyrrolyl)alanine, 1-, 3-, or 4-pyrazolylalanine,

$R'_4$ is hydrogen or ($C_1$-$C_6$)-alkyl, preferably hydrogen and

T is a mimic of the Leu-Val cleavage site of angiotensinogen,

or a pharmaceutically acceptable salt thereof.

**2.** The compound as claimed in claim 1, in which

$R^A$ and $R^B$ are the same or different and are hydrogen, ($C_1$-$C_4$)-alkyl, ($C_4$-$C_6$)-cycloalkyl, $C_4$-$C_6$)-cycloalkyl-($C_1$-$C_4$)-alkyl, ($C_6$-$C_{12}$)-aryl, which may be substituted by one or two identical or different residues of the group methyl, ethyl, methoxy, ethoxy, halogen or amino,

partially hydrogenated ($C_6$-$C_{12}$)-aryl,

($C_6$-$C_{12}$)-aryl-($C_1$-$C_4$)-alkyl, which may be substituted in the aryl part as defined above, ($C_1$-$C_6$)-alkanoyl,

($C_6$-$C_{12}$)-aryl-($C_1$-$C_4$)-alkanoyl, which may be substituted in the aryl part as defined above,
($C_4$-$C_{10}$)-hetaryl-($C_1$-$C_4$)-alkanoyl,
($C_1$-$C_4$)-alkoxy-($C_1$-$C_4$)alkanoyl,
($C_1$-$C_4$)-alkoxycarbonyl-($C_1$-$C_6$)-alkanoyl,
($C_6$-$C_{12}$)-aryloxycarbonyl-($C_1$-$C_6$)-alkanoyl, which may be substituted in the aryl part as defined above,
($C_6$-$C_{12}$)-aryl-($C_1$-$C_4$)-alkoxycarbonyl, which may be substituted in the aryl part as defined above or in which

$R^A$ and $R^B$ form together with the N-atom bearing them a piperidino-, pyrrolidino-, morpholino-, piperazine or thiomorpholino-ring; or in which

$R^A$ is defined as above and

$R^B$ is amino, methylamino, ethylamino, di-($C_1$-$C_2$-alkylamino, hydroxy, methoxy, ethoxy, methylsulfonyl, ethylsulfonyl, phenylsulfonyl or carbamoyl;

$R^F$ is defined as for $R^A$ and $R^B$ above;

$R^G$ is hydrogen, ($C_1$-$C_3$)-alkyl, ($C_4$-$C_6$)-cycloalkyl, ($C_4$-$C_6$)-cycloalkyl-($C_1$-$C_2$)-alkyl, ($C_6$-$C_{12}$)-aryl or ($C_6$-$C_{12}$)-aryl-($C_1$-$C_2$)-alkyl;

$R^C$, $R^D$ and $R^E$ are same or different and are hydrogen or ($C_1$-$C_3$)-alkyl or $R^D$ and $R^E$ form an ethylene bridge;

h is 0, 1 or 2;
i is 0, 1 or 2;
k is 1, 2 or 3;
l is 1, 2 or 3;
X is -CO- or -$SO_2$-;
Y is $(CH_2)_q$-$(CR^HR^L)_r$ or -O-, wherein
q is 0, 1 or 2,
r is 0, 1 or 2,

$R^H$ and $R^L$ are the same or different and are hydrogen or $(C_1-C_3)$-alkyl;
Z is $-CH_2-$ or absent and
all other residues are defined as above.

**3.** The compound as claimed in claim 1 or 2, in which
$R^A$, $R^B$ and $R^F$ are the same or different and are hydrogen, $(C_1-C_4)$-alkyl, $(C_1-C_4)$-alkanoyl, e.g. acetyl, $(C_6-C_{12})$-aryl-$(C_1-C_4)$-alkyl, e.g. benzyl; $(C_7-C_{13})$-aroyl, e.g. benzyl, $(C_4-C_{10})$-hetaroyl, e.g. nicotinoyl, $(C_1-C_4)$-alkoxycarbonyl, e.g. t.-butoxycarbonyl, or benzyloxycarbonyl;

| | |
|---|---|
| $R^G$ | is hydrogen or methyl; |
| $R^C$, $R^D$ and $R^E$ | are the same or different and are hydrogen, methyl or ethyl or |
| $R^D$ and $R^E$ | form an ethylene bridge; |
| h | is 0, 1 or 2; |
| i | is 0, 1 or 2; |
| k | is 1, 2 or 3; |
| l | is 1, 2 or 3; |
| X | is -CO- or $SO_2$-; |
| Y | is $-CH_2-$ or -O-; |
| Z | is $-CH_2-$ or absent; |
| $R_2$ | is cyclohexylmethyl, benzyl, 1- or 2-naphthylmethyl, 2-,3- or 4-thienylmethyl, p-methoxybenzyl, p-fluorobenzyl, most preferentially benzyl and in which all other residues are defined as above. |

**4.** A process for preparation of compounds of formula I as claimed in any of claims 1 to 3, wherein a fragment with a C-terminal carboxy group or its reactive derivative is reacted with another fragment with a free N-terminal amino group, temporarily introduced protecting groups are optionally removed and the compounds so obtained are optionally transferred into their physiologically acceptable salts.

**5.** The compound as claimed in any of claims 1 to 4 for the use a medicament.

**6.** A pharmaceutical composition comprising an effective amount of at least one of a compound as claimed in any of claims 1 to 3 and 5, and a pharmaceutically acceptable carrier.

European Patent Office

# EUROPEAN SEARCH REPORT

Application Number

EP 90 12 0882

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X,Y | EP-A-0 339 483 (MERCK PATENT GmbH)<br>* Page 13, lines 29-32; page 14, lines 9-24; examples 9,15,17; claims 1-7 * | 1-6 | C 07 D 401/12<br>C 07 D 413/14<br>C 07 D 405/14<br>C 07 D 211/06<br>A 61 K 31/445 |
| X,Y | EP-A-0 309 481 (MERCK PATENT GmbH)<br>* Page 27, lines 39-53; page 28, lines 28-39; page 29, lines 1-30; page 30, lines 1-40; claims 1-7 * | 1-6 | |
| Y | EP-A-0 371 390 (MERCK PATENT GmbH)<br>* Examples 1,3; claims 1-7 * | 1-6 | |
| Y | EP-A-0 337 334 (MERCK PATENT GmbH)<br>* Examples 3,5,6,9,11,17,18; claims 1-7 * | 1-6 | |
| Y | EP-A-0 200 406 (KISSEI PHARM. CO.)<br>* The whole document, especially column 5, lines 47-53 * | 1-6 | |
| Y | BIOCHEMICAL AND BIOPHYSICAL RESEARCH COMMUNICATIONS, vol. 160, no. 1, 14th April 1989, pages 1-5, Academic Press Inc.; G.J HANSON et al.: "A new class of orally active glycol renin inhibitors containing phenyllactic acid at P3"<br>* The whole article * | 1-6 | TECHNICAL FIELDS SEARCHED (Int. Cl.5)<br>A 07 D<br>A 61 K |
| A | PROCEEDINGS OF THE TENTH AMERICAN PEPTIDE SYMPOSIUM, 23rd-28th May 1987, pages 474-475, St. Louis, Missouri, US; D.J. KEMPF et al.: "Renin inhibitors based on novel dipeptide analogs: Increased efficacy through systematic inclusion of polar functionality"<br>* The whole article * | 1-6 | |

~~The present search report has been drawn up for all claims~~

| Place of search | Date of completion of the search | Examiner |
|---|---|---|
| THE HAGUE | 27-06-1991 | GROENENDIJK M.S.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

EP 90 12 0882

## CLAIMS INCURRING FEES

The present European patent application comprised at the time of filing more than ten claims.

☐ All claims fees have been paid within the prescribed time limit. The present European search report has been drawn up for all claims.

☐ Only part of the claims fees have been paid within the prescribed time limit. The present European search report has been drawn up for the first ten claims and for those claims for which claims fees have been paid,

namely claims:

☐ No claims fees have been paid within the prescribed time limit. The present European search report has been drawn up for the first ten claims.

## LACK OF UNITY OF INVENTION

The Search Division considers that the present European patent application does not comply with the requirement of unity of invention and relates to several inventions or groups of inventions,
namely:

See sheet -B-

☐ All further search fees have been paid within the fixed time limit. The present European search report has been drawn up for all claims.

☐ Only part of the further search fees have been paid within the fixed time limit. The present European search report has been drawn up for those parts of the European patent application which relate to the inventions in respect of which search fees have been paid,

namely claims:

☒ None of the further search fees has been paid within the fixed time limit. The present European search report has been drawn up for those parts of the European patent application which relate to the invention first mentioned in the claims,

namely claims: 1-6 (part.)

European Patent Office

## LACK OF UNITY OF INVENTION

The Search Division considers that the present European patent application does not comply with the requirement of unity of invention and relates to several inventions or groups of inventions,

namely:

1. Claims 1-6 (partially): Compounds of formula I where in R1 is a residu of formula II, their preperation and pharmaceutical compositions containing them.

2. Claims 1-6 (partially): Compounds of formula I where in R1 is a residu of formula III, then preperation and pharmaceutical compositions containing them.

Claim 1 of the present application relates to compounds with renin inhibiting activity which contain at the N-terminal azacyclic aryl or aminoaryl residues.
Besides the alpha-nitrogen of the amino acid at the $P_3$-location has been replaced by O,C or S.
The object of these modifications is to obtain a better absorption and a longer duration of action in vivo.
At the data of filing of the present application compounds covered by the formula of claim 1, wherein this modifications have been made for the same reasons where already known (e.g. EP 339483). For this reason no unifying inventive concept can be acknowledged for the compounds covered by claim 1.
As distinct inventive concepts can be acknowledged the introduction of the residues II and III in the compounds of the general type of formula I.